(19) Europäisches Patentamt
European Patent Office
Office européen des brevets

(11) EP 4 763 817 A2

# (12) EUROPEAN PATENT APPLICATION

(43) Date of publication:
24.06.2026 Bulletin 2026/26

(51) International Patent Classification (IPC):
C03C 4/08 (2006.01)

(21) Application number: 26160784.0

(22) Date of filing: 01.06.2020

(52) Cooperative Patent Classification (CPC):
C03C 12/00; A61K 49/0419; A61K 51/1244;
A61L 24/001; A61L 24/0015; A61L 24/02;
C03C 3/095; C03C 4/087; A61L 2300/44;
A61L 2400/06; A61L 2430/36; A61M 5/00

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR

(30) Priority: 31.05.2019 US 201962855285 P

(62) Document number(s) of the earlier application(s) in accordance with Art. 76 EPC:
20813381.9 / 3 976 542

(71) Applicant: ABK Biomedical Incorporated
Halifax, Nova Scotia B3S 1B3 (CA)

(72) Inventors:
• LEGERE, Sharon
Bedford, B4B 0T2 (CA)
• BOYD, Daniel
Run Upper Tantallon, B3Z 0B2 (CA)
• O'CONNELL, Kathleen
B1N 3J2 South Bar (CA)
• GREGOIRE, Marc
K1S 0M4 Ottowa (CA)
• HEADLEY, Jr. F. Anthony
30642 Greensboro (US)

(74) Representative: Potter Clarkson
Chapel Quarter
Mount Street
Nottingham NG1 6HQ (GB)

Remarks:
• This application was filed on 25-02-2026 as a divisional application to the application mentioned under INID code 62.
• Claims filed after the date of filing of the application (Rule 68(4) EPC).

## (54) RADIOPAQUE GLASS MATERIAL

(57) A glass material that includes: from about 0.55 to about 0.85 mole fraction of $SiO_2$; from about 0.01 to about 0.23 mole fraction of $Na_2O$, $K_2O$, or a combination of $Na_2O$ and $K_2O$; from about 0.05 to about 0.28 mole fraction of: $Y_2O_3$, BaO, or a combination of $Y_2O_3$ and BaO; and optionally $Ta_2O_5$. In the glass material, the sum of the $Y_2O_3$, the BaO and the optional $Ta_2O_5$ is from about 0.10 to about 0.31 mole fraction. The glass material may be in the form of microspheres. The microspheres may be used for vascular embolization and/or radiologic imaging.

Fig. 1

EP 4 763 817 A2

**Description**

**CROSS-REFERENCE TO RELATED APPLICATIONS**

This application claims priority to U.S. Provisional Patent Application No. 62/855,285, which was filed on May 31, 2019, the entire contents of which is incorporated herein by reference.

**FIELD**

The present disclosure relates to radiopaque glass material suitable for forming into microparticles that are administrable to a patient.

**BACKGROUND**

The following paragraph is not an admission that anything discussed in it is prior art or part of the knowledge of persons skilled in the art.
Therapeutic vascular occlusions (embolizations) are techniques used to treat certain pathological conditions *in situ.* Therapeutic embolization is practiced generally using a catheter to position embolization agents in the circulatory system, such as the vessels of various processes: tumors, vascular malformations, and hemorrhagic processes.

**INTRODUCTION**

The following introduction is intended to introduce the reader to this specification but not to define any invention. One or more inventions may reside in a combination or sub-combination of the apparatus elements or method steps described below or in other parts of this document. The inventors do not waive or disclaim their rights to any invention or inventions disclosed in this specification merely by not describing such other invention or inventions in the claims.
Particulate embolic agents used in vascular embolization may not be radiopaque, and are therefore difficult to view with radiologic imaging, unless they are treated with a contrast agent prior to injection. TheraSphere™ yttrium-90 microspheres, a commercially available radioactive microsphere used to treat primary and metastatic liver cancer, has a radiopacity of about 6,000 Hounsfield Units (HU) at 120 kVp.
Radiopaque particulate embolic agents are desirable since they may be viewed with radiologic imaging during or after the embolization treatment. One or more examples described in the present disclosure attempt to provide a radiopaque glass material having a radiopacity greater than otherwise comparable particulate embolic agents.
Glass material that is ultimately intended to be used in vascular embolization may have a density from about 2.7 $g/cm^3$ to about 4.3 $g/cm^3$. In particular examples, the glass material may have a density from about 2.9 $g/cm^3$ to about 3.7 $g/cm^3$, or from about 3.3 $g/cm^3$ to about 3.6 $g/cm^3$. Particles with a density in this range are believed to appropriately distribute within the vasculature of a patient.
In the glass material of the present disclosure, $Y_2O_3$, BaO and $Ta_2O_5$ all contribute to the radiopacity of the glass. However, increasing the amount of these components also increases the density of the resulting glass material. The authors of the present disclosure have identified glass compositions that provide a desirable amount of radiopacity at densities suitable for vascular embolization. Some glass material of the present disclosure may have a radiopacity of more than 9,000 HU at 120 kVp. Some glass material of the present disclosure may have a density from about 2.7 $g/cm^3$ to about 4.3 $g/cm^3$, such as from about 2.9 $g/cm^3$ to about 3.7 $g/cm^3$, or from about 3.3 $g/cm^3$ to about 3.6 $g/cm^3$.
In one aspect, the present disclosure provides a glass material that includes: from about 0.55 to about 0.85 mole fraction of $SiO_2$; from about 0.01 to about 0.23 mole fraction of $Na_2O$, $K_2O$, or a combination of $Na_2O$ and $K_2O$; from about 0.05 to about 0.28 mole fraction of: $Y_2O_3$, BaO, or a combination of $Y_2O_3$ and BaO; and optionally $Ta_2O_5$. In the glass material, the sum of the $Y_2O_3$, the BaO and the optional $Ta_2O_5$ is from about 0.10 to about 0.31 mole fraction.
In some examples, the glass material includes from about 0.03 to about 0.23, such as from about 0.05 to about 0.23, mole fraction of $Na_2O$, $K_2O$, or a combination of $Na_2O$ and $K_2O$.
In some examples, the glass material includes from about 0.55 to about 0.82, such as from about 0.55 to about 0.80, mole fraction of $SiO_2$.
In a particular example, the glass material includes: from about 0.55 to about 0.80 mole fraction of $SiO_2$; from about 0.05 to about 0.22 mole fraction of $Na_2O$, $K_2O$, or a combination of $Na_2O$ and $K_2O$.
In one particular example, the glass material includes about 0.74 mole fraction of $SiO_2$; about 0.09 mole fraction of $Na_2O$; about 0.085 mole fraction of BaO; about 0.085 mole fraction of $Ta_2O_5$; and about 0.006 mole fraction of $B_2O_3$. This particular glass material exhibits a radiopacity of about 16,000 HU at 120 kVp.
In another particular example, the glass material includes about 0.79 mole fraction of $SiO_2$; about 0.06 mole fraction of $Na_2O$; about 0.08 mole fraction of BaO; about 0.07 mole fraction of $Ta_2O_5$; and about 0.003 mole fraction of

$B_2O_3$.

In yet another particular example, the glass material includes about 0.77 mole fraction of $SiO_2$; about 0.031 mole fraction of $Na_2O$; about 0.10 mole fraction of $BaO$; about 0.096 mole fraction of $Ta_2O_5$; and about 0.002 mole fraction of $B_2O_3$.

In still yet another particular example, the glass material includes about 0.80 mole fraction of $SiO_2$; about 0.033 mole fraction of $Na_2O$; about 0.82 mole fraction of $BaO$; about 0.085 mole fraction of $Ta_2O_5$; and about 0.001 mole fraction of $B_2O_3$.

In some examples of the present disclosure, the glass material of the present disclosure is a bulk glass. The term "bulk glass" refers to glass material obtained by forming a glass from the starting reagents without any further processing steps to make the glass material suitable for vascular embolization. For example, glass material produced on a commercial scale without any processing steps to make irregular microparticulate glass material may be considered bulk glass.

In other examples, the glass material of the present disclosure is an irregular microparticulate glass material. The term "irregular microparticulate glass material" refers to particulate material that is sized, but not appropriately shaped, for vascular embolization. Irregular microparticulate glass material may be prepared by pulverizing bulk glass, and sieving the resulting particles to retrieve microparticles of a desired size.

In still other examples, the glass material of the present disclosure is a substantially spherical microparticulate glass material. The term "substantially spherical microparticulate glass material" refers to particulate material that is sized and shaped for vascular embolization. Substantially spherical microparticulate glass material may be prepared by re-melting the surface of irregular microparticulate glass material, and allowing a substantially spherical drop to form.

In another aspect, a method is provided for making glass material according to the present disclosure.

In still another aspect of the present disclosure, substantially spherical microparticulate glass material as described herein may be used for an X-ray based radiologic imaging technique, such as radiography imaging, computerized tomography (CT) imaging, cone beam CT imaging, or fluoroscopy imaging. The present disclosure also provides a method of imaging a mammal using substantially spherical microparticulate glass material as described herein.

In vascular embolization treatments that use radioactive microparticles, it is believed that administering more microparticles with a lower specific activity is desirable because increasing the number of microparticles results in better tumour coverage in comparison to administering fewer microparticles at a higher specific activity. Without wishing to be bound by theory, the authors of the present disclosure believe that microparticles come to rest with the first available localization spots in the vasculature that they encounter. Administering a smaller number of microparticles may concentrate some of the particles in one portion of the tumour if there are sufficient localization spots to interact with a significant portion of the administered particles. In contrast, administering a larger number of lower activity particles better saturates more of the available localization spots and leads to more uniform coverage in the tumour.

For at least some tumor sizes and/or degrees of vascularization, administering to the patient a mixture of (i) radioactive microparticles; and (ii) radiopaque non-radioactive microparticles according to the present disclosure may provide at least some of the benefits associated with administering more microparticles at a lower specific activity, even if the individual radioactive microparticles are at a higher specific activity.

In one aspect, the present disclosure provides a mixture of (i) radioactive glass microparticles; and (ii) non-radioactive, radiopaque microparticulate glass material according to the present disclosure, where the radioactive glass microparticles are suitable to treat tumors in the liver, and where the radioactive glass microparticles and the non-radioactive radiopaque microparticulate glass material have substantially the same size. In particular examples, the microparticulate glass material of the present disclosure and the radioactive glass microparticles have substantially the same density.

In some examples, the mixture may be prepared by exposing pre-radioactive glass microparticles to neutron activation to form the radioactive glass microparticles, and combining the radioactive glass microparticles with the radiopaque microparticulate glass material of the present disclosure.

In one particular example, the mixture includes (i) substantially spherical radioactive yttrium oxide-aluminosilicate glass microparticles comprising about 40 wt% $SiO_2$, about 20 wt% $Al_2O_3$, and about 40 wt% $Y_2O_3$ (which is equivalent to about 0.170 mole fraction $Y_2O_3$, about 0.189 mole fraction $Al_2O_3$, and about 0.641 mole fraction $SiO_2$); and (ii) substantially spherical microparticulate glass material that include from about 0.73 to about 0.80 mole fraction of $SiO_2$; from about 0.03 to about 0.11 mole fraction of $Na_2O$; from about 0.07 to about 0.10 mole fraction of $BaO$; from about 0.07 to about 0.10 mole fraction of $Ta_2O_5$; and from about 0.001 to about 0.006 mole fraction of $B_2O_3$.

In still another aspect of the present disclosure, radiation is delivered to a mammal by administering the mixture to the mammal when the mixture includes radioactive glass microspheres. Such a method may additionally include imaging the mammal using an X-ray based radiologic imaging technique, in particular using a static imaging technique. The imaging technique may include fluoroscopy, Computed Tomography/Positron Emission Tomography (CT/PET) or Cone Beam Computed Tomography (CBCT).

Glass material according to the present disclosure may be included in compositions or delivery devices, or used in diagnostic or therapeutic methods.

In some aspects, the present disclosure provides a therapeutic or diagnostic composition that includes a mixture of radioactive microparticles; and non-radioactive microparticles where at least some of the non-radioactive microparticles include the glass material disclosed herein.

In other aspects, the present disclosure provides a method that includes administering the therapeutic or diagnostic composition to a patient, where the administration is: by intravascular delivery, intra-peritoneal delivery, or percutaneous delivery.

In one aspect, the present disclosure provides a delivery device for intravascular delivery, intra-peritoneal delivery, or percutaneous delivery of a mixture of radioactive microparticles and non-radioactive microparticles to a patient. The delivery device is fluidly coupleable to a mixing and transport medium. The delivery device includes a fluid inlet fluidly coupleable to the mixing and transport medium; a fluid outlet; a fluid mixer fluidly coupled to the fluid inlet and to the fluid outlet; a source of radioactive microparticles fluidly coupled to the fluid mixer; and a source of non-radioactive microparticles fluidly coupled to the fluid mixer. At least some of the non-radioactive microparticles are composed of glass material according to the present disclosure. The source of the radioactive microparticles is distinct from the source of non-radioactive microparticles. The fluid mixer mixes the radioactive microparticles with the non-radioactive microparticles, and delivers the mixture of radioactive and non-radioactive microparticles out of the fluid outlet utilizing the mixing and transport medium.

In another aspect, the present disclosure provides a delivery device for intravascular delivery, intra-peritoneal delivery, or percutaneous delivery of a mixture of radioactive microparticles and non-radioactive microparticles to a patient. The delivery device includes: at least one fluid inlet fluidly coupleable to a transport medium; a source of radioactive microparticles fluidly coupled to the at least one fluid inlet; a source of non-radioactive microparticles fluidly coupled to the at least one fluid inlet; a first fluid outlet fluidly coupled to the source of the radioactive microparticles; and a second fluid outlet fluidly coupled to the source of non-radioactive microparticles. At least some of the non-radioactive microparticles are composed of glass material according to the present disclosure. The source of the radioactive microparticles is distinct from the source of non-radioactive microparticles.

In the context of the present disclosure, it should be understood that one population of microparticles is distinct from another population of microparticles if the two populations are not mixed together. For example, radioactive microparticles in the barrel of one syringe would be considered to be distinct from non-radioactive microparticles in the barrel of a second syringe even if the two syringes were fluidly coupled together and capable of expelling the microparticles together to form a mixture.

In still another aspect, the present disclosure provides a method that includes mixing (i) a first population of radioactive microparticles and (ii) a second population of non-radioactive microparticles, and administering a therapeutically or diagnostically relevant amount of the mixture to a patient. At least some of the non-radioactive microparticles are composed of glass material according to the present disclosure.

In yet another aspect, the present disclosure provides a method of administering a therapeutically or diagnostically relevant amount of microparticles to a patient. The method includes: administering non-radioactive microparticles to the patient; and administering radioactive microparticles to the patient without first detecting the non-radioactive microparticles. At least some of the non-radioactive microparticles are composed of glass material according to the present disclosure. The administration is by intravascular delivery, intra-peritoneal delivery, or percutaneous delivery; and the route of administration of the non-radioactive microparticles is the same as the route of administration of the radioactive microparticles.

In yet another aspect, the present disclosure provides a method of administering a therapeutically or diagnostically relevant amount of microparticles to a patient. The method includes: administering radioactive microparticles to the patient; and administering non-radioactive microparticles to the patient without first detecting the radioactive microparticles. At least some of the non-radioactive microparticles are composed of glass material according to the present disclosure. The administration is by intravascular delivery, intra-peritoneal delivery, or percutaneous delivery; and the route of administration of the non-radioactive microparticles is the same as the route of administration of the radioactive microparticles.

In still another aspect, the present disclosure provides a method of administering a therapeutically or diagnostically relevant amount of microparticles. The method includes: concurrent administration of (i) a first population of radioactive microparticles and (ii) a second population of non-radioactive microparticles to a patient. At least some of the non-radioactive microparticles are composed of glass material according to the present disclosure.

In still another aspect, the present disclosure provides a method of administering a therapeutically or diagnostically relevant amount of microparticles. The method includes: sequential administration in a single treatment session of non-radioactive microparticles, and of radioactive microparticles to a patient. At least some of the non-radioactive microparticles are composed of glass material according to the present disclosure.

In yet another aspect, the present disclosure provides a method that includes sequential administration to a patient of (i) therapeutically radioactive microparticles, and then (ii) non-radioactive microparticles. At least some of the non-radioactive microparticles are composed of glass material according to the present disclosure.

In any of the aspects discussed above, the non-radioactive microparticles may be any of the non-radioactive glass compositions discussed in the section below entitled "Glass compositions"; and/or may have any of the features, alone or in combination, of the glass materials discussed in the section below entitled "Glass materials". In any of the aspects discussed above, the radioactive microparticles may be any of the radioactive glass compositions discussed in the section below related to radiotherapeutic mixtures.

## BRIEF DESCRIPTION OF THE DRAWINGS

Embodiments of the present disclosure will now be described, by way of example only, with reference to the attached Figures.

Fig. 1 is a graph illustrating the cumulative ion release curve obtained for an exemplary glass composition (microspheres, post-spherodization) of the present disclosure subjected to extraction in calcium- and magnesium-free phosphate buffered saline (CMF-PBS).

Fig. 2A is a set of SEM images at 250x and 1000x of irregular BIC2 glass powder.

Fig. 2B is a set of SEM images at 250x and 1000x of post-spherodized BIC2 microspheres.

Fig. 2C is a set of SEM images at 250x and 1000x of post-spherodized and post conditioned (24 hours), re-sieved, BIC2 microspheres.

Fig. 2D is a set of SEM images at 250x and 1000x of post-spherodized and post conditioned (72 hours), re-sieved, BIC2 microspheres.

Fig. 3 is a graph illustrating the CT Radiography for various glass microspheres as a function of volume fraction.

Fig. 4 is a graph illustrating the R1 Relaxivity for various glass microspheres as a function of volume fraction.

Fig. 5 is an image of representative T1-weighted, T2-weighted and T2*-weighted BIC2 and COMP1 articles at varied fractional volume.

Fig. 6 is a graph illustrating the R2 Relaxivity for various glass microspheres as a function of volume fraction.

Fig.7 is a graph illustrating the R2* Relaxivity for various glass microspheres as a function of volume fraction.

Fig. 8 is a graph illustrating the Local Magnetic Dose (LMD) relaxivity obtained MRI Susceptometry measurements for various glass microspheres as a function of volume fraction.

## DETAILED DESCRIPTION

In the context of the present disclosure, it should be understood that "glass material" generally refers to physical material, such as bulk or microparticulate material, that includes glass of the specified composition. The term "glass" or "glass composition" defines the specific components of the composition. Accordingly, reference to physical properties of a material (e.g. particle size) relates to the glass material, while reference to compositional properties (e.g. mole fractions) relates to the glass or glass composition. In some portions of this disclosure, the terms "glass", "glass composition" and "glass material" are used interchangeably, such as if they all refer to the same component, for example if a glass material is made up only of the noted glass composition.

In the context of the present disclosure, any disclosed range of values should be interpreted in a flexible manner to include not only the numerical values explicitly recited as the limits of the range, but also to include all the individual numerical values or subranges encompassed within that range as if each numerical value and sub-range is explicitly recited. For example, a range of "about 1 to about 10" should be interpreted to include not just about 1 to about 10, but also the individual values (e.g., 1, 1.5, 2, 4 ... etc.) and the sub-ranges (e.g., 1 to 3, 2 to 7, 5 to 6, 2 to 10, etc.) within the disclosed range of values.

**Glass compositions.** Generally, the present disclosure provides a glass material where the glass includes: from about 0.55 to about 0.85 mole fraction of $SiO_2$; and from about 0.01 to about 0.23 mole fraction of $Na_2O$, $K_2O$, or a combination of $Na_2O$ and $K_2O$. Since the amounts of $Y_2O_3$, BaO and $Ta_2O_5$ in a glass all affect the radiopacity and the density of a glass, and may additionally affect the ability of the other components to form a glass, the acceptable amounts of $Y_2O_3$, BaO and/or $Ta_2O_5$ are not predictable *a prior.* The authors of the present disclosure have determined, based on experimental results, that the glass material of the present disclosure must include from about 0.05 to about 0.28 mole fraction of: $Y_2O_3$, BaO, or a combination of $Y_2O_3$ and BaO; and optionally $Ta_2O_5$; and that the sum of the $Y_2O_3$, the BaO and the optional $Ta_2O_5$ is from about 0.10 to about 0.31 mole fraction. In some examples, the sum of the $Y_2O_3$, the BaO and the optional $Ta_2O_5$ may be from about 0.10 to about 0.25 mole fraction, such as from about 0.10 to about 0.15 mole fraction.

In the context of mole fractions, it should be understood that "about X mol fraction" refers to any value that is within the estimated uncertainty associated with the noted value and the oxide being measured. For example, in the context of inductively coupled plasma mass spectrometry (ICP-MS): the estimated uncertainty of $SiO_2$ may be 1% of the reported value; the estimated uncertainty of $Na_2O$ may be 10% of the noted value; the estimated uncertainty of BaO may be 2% of the reported value; and the estimated uncertainty of $Ta_2O_5$ may be 1% of the reported value.

The glass of the present disclosure may include from about 0.05 to about 0.08 mole fraction of $Y_2O_3$, BaO, or a combination of $Y_2O_3$ and BaO. Some exemplary glasses of the present disclosure do not include any $Y_2O_3$. Such exemplary glasses may be useful in mixtures of microspheres, as discussed in greater detail below.

The glass of the present disclosure may include $Ta_2O_5$ in an amount up to about 0.12, for example up to 0.10, mole fraction of $Ta_2O_5$, such as from about 0.05 to about 0.12, such as from 0.05 to about 0.10, mole fraction of $Ta_2O_5$.

The glass of the present disclosure may additionally include $B_2O_3$ in an amount up to 0.20 mole fraction of $B_2O_3$. The sum of the $B_2O_3$ and the $SiO_2$ may be from about 0.60 to about 0.85, such as from about 0.60 to about 0.80, mole fraction. $B_2O_3$ is known in the art as a glass former, and the inclusion of $B_2O_3$ in a sodium silicate glass results in a sodium borosiliate glass. Some examples of sodium borosiliate glasses of the present disclosure may be more resistant to degradation. Some examples of sodium borosiliate glasses of the present disclosure may have an improved glass forming ability.

Although the present disclosure focuses on sodium silicate glasses, both $Na_2O$ and $K_2O$ are known to act as glass network modifiers with many similar effects on glass properties. In some contemplated glasses of the present disclosure, the glass composition may include only $Na_2O$ (without any $K_2O$), or only $K_2O$ (without any $Na_2O$). In other contemplated glasses, the glass composition may include $Na_2O$ and $K_2O$ in a ratio from 100:1 to 1:100. The present disclosure also contemplates any ratio therebetween, such as ratios of 50:1, 25:1, 10:1, 5:1, 1:1, 1:5, and 1:10, and any range of ratios therebetween. In some examples of the present disclosure, the ratio of Na:K may be about 75:25, about 50:50, about 25:75, or about 10:90.

In addition to affecting the radiopacity of the glass, BaO is also known in the art to act as a glass network modifier. If the total amount of network modifiers in a sodium silicate glass is too large, then the glass may not form or may be unsuitable as a permanent embolic agent due to instability of the glass network. In a glass material of the present disclosure that includes BaO, the sum of BaO, $Na_2O$ and $K_2O$, may be from about 0.10 to about 0.33, such as from about 0.15 to about 0.33, mole fraction. For example, the sum of the BaO, $Na_2O$ and $K_2O$ may be from about 0.10 to about 0.25, for example from about 0.15 to about 0.25, mole fraction.

The glass of the present disclosure is preferably predominantly a sodium silicate glass. The sum of the $SiO_2$ and the $Na_2O$ in the glass of the present disclosure may be from about 0.65 to about 0.90 mole fraction.

In some examples of the glass material disclosed herein, the sum of the $SiO_2$ and the $Na_2O$ is from about 0.69 to about 0.90 mole fraction; the glass includes from about 0.05 to about 0.09 mole fraction of $Ta_2O_5$; and the sum of the $Y_2O_3$, the BaO and the $Ta_2O_5$ is from about 0.10 to about 0.17 mole fraction. Exemplary glass material with such a composition may exhibit a beneficial balance between high levels of radiopacity and particle densities from 3.3 to 3.6 g/cm$^3$.

In other examples of the glass material disclosed herein, the sum of the $SiO_2$ and the $Na_2O$ is from about 0.65 to about 0.80 mole fraction; the glass includes from about 0.05 to about 0.10 mole fraction of $Ta_2O_5$; and the sum of the $Y_2O_3$, the BaO and the $Ta_2O_5$ is from about 0.18 to about 0.31 mole fraction. Exemplary glass material with such a composition may exhibit a high level of radiopacity, though at an increased density.

In still other examples of the glass material disclosed herein, the glass includes from about 0.55 to about 0.80, such as from about 0.55 to about 0.75, mole fraction of $SiO_2$; from about 0.03 to about 0.23, such as from about 0.05 to about 0.22, mole fraction of $Na_2O$; from about 0.05 to about 0.12, such as from about 0.05 to about 0.08, mole fraction of: $Y_2O_3$, BaO, or a combination of $Y_2O_3$ and BaO; and from about 0.05 to about 0.12, such as from about 0.05 to about 0.09, mole fraction of $Ta_2O_5$.

For example, the glass may include: from about 0.70 to about 0.73 mole fraction of $SiO_2$; from about 0.16 to about 0.18 mole fraction of $Na_2O$; from about 0.05 to about 0.7 mole fraction of $Y_2O_3$; and from about 0.05 to about 0.7 mole fraction of $Ta_2O_5$. One specific example is a glass that includes about 0.71 mole fraction of $SiO_2$; about 0.17 mole fraction of $Na_2O$; about 0.06 mole fraction of $Y_2O_3$; and about 0.06 mole fraction of $Ta_2O_5$. Another specific example is a glass that includes about 0.72 mole fraction of $SiO_2$; about 0.17 mole fraction of $Na_2O$; about 0.05 mole fraction of $Y_2O_3$; and about 0.06 mole fraction of $Ta_2O_5$.

In some examples of the glass material disclosed herein, the glass includes from about 0.55 to about 0.82, such as from about 0.55 to about 0.75, mole fraction of $SiO_2$; from about 0.03 to about 0.23, such as about 0.05 to about 0.22, mole fraction of $Na_2O$; from about 0.05 to about 0.12, such as about 0.05 to about 0.08, mole fraction of: $Y_2O_3$, BaO, or a combination of $Y_2O_3$ and BaO; from about 0.05 to about 0.12, such as about 0.05 to about 0.09, mole fraction of $Ta_2O_5$; and up to 0.1 mole fraction of $B_2O_3$.

In some examples, the glass includes from about 0.65 to about 0.82, such as about 0.73 to about 0.80, mole fraction of $SiO_2$; from about 0.03 to about 0.23, such as about 0.03 to about 0.11, mole fraction of $Na_2O$; from about 0.06 to about 0.12, such as about 0.07 to about 0.10, mole fraction of BaO; from about 0.06 to about 0.12, such as about 0.07 to about 0.10, mole fraction of $Ta_2O_5$; and from about 0.001 to about 0.015, such as about 0.001 to about 0.006, mole fraction of $B_2O_3$.

One specific example is a glass that includes: about 0.69 mole fraction of $SiO_2$; about 0.16 mole fraction of $Na_2O$; about 0.07 mole fraction of BaO; about 0.07 mole fraction of $Ta_2O_5$; and about 0.01 mole fraction of $B_2O_3$.

Another specific example is a glass that includes: about 0.66 mole fraction of $SiO_2$; about 0.20 mole fraction of

$Na_2O$; about 0.065 mole fraction of BaO; about 0.06 mole fraction of $Ta_2O_5$; and about 0.011 mole fraction of $B_2O_3$.

Another specific example is a glass that includes: about 0.73 mole fraction of $SiO_2$; about 0.10 mole fraction of $Na_2O$; about 0.08 mole fraction of BaO; about 0.08 mole fraction of $Ta_2O_5$; and about 0.01 mole fraction of $B_2O_3$.

Still another specific example is a glass that includes: about 0.74 mole fraction of $SiO_2$; about 0.09 mole fraction of $Na_2O$; about 0.085 mole fraction of BaO; about 0.085 mole fraction of $Ta_2O_5$; and about 0.006 mole fraction of $B_2O_3$.

Yet another specific example is a glass that includes: about 0.79 mole fraction of $SiO_2$; about 0.06 mole fraction of $Na_2O$; about 0.08 mole fraction of BaO; about 0.07 mole fraction of $Ta_2O_5$; and about 0.003 mole fraction of $B_2O_3$.

A further specific example is a glass that includes: about 0.77 mole fraction of $SiO_2$; about 0.031 mole fraction of $Na_2O$; about 0.10 mole fraction of BaO; about 0.096 mole fraction of $Ta_2O_5$; and about 0.002 mole fraction of $B_2O_3$.

A still further specific example is a glass that includes: about 0.80 mole fraction of $SiO_2$; about 0.033 mole fraction of $Na_2O$; about 0.082 mole fraction of BaO; about 0.085 mole fraction of $Ta_2O_5$; and about 0.001 mole fraction of $B_2O_3$.

A further example is a glass that includes: about 0.58 mole fraction of $SiO_2$; about 0.20 mole fraction of $Na_2O$; about 0.06 mole fraction of a combination of $Y_2O_3$ and BaO; about 0.07 mole fraction of $Ta_2O_5$; and about 0.09 mole fraction of $B_2O_3$. The glass may include about 0.024 mole fraction of $Y_2O_3$ and about 0.035 mole fraction of BaO.

Glass compositions according the present disclosure are preferably substantially lacking in one or more of $Li_2O$, $Rb_2O$, $V_2O_5$, ZnO, $FeO_2$, and fluoride. The lithium, rubidium and vanadium oxides may make the glass cytotoxic if those oxides are present in sufficiently high concentrations and if those oxides are capable of leaching out of the glass material. It is desirable to keep these oxides below 0.01 mole fraction, such as at 0.001 or 0.0001 mole fraction, and preferably at 0 mole fraction. Fluoride in the glass increases degradation under physiological conditions. It is desirable to keep fluoride below 0.005 mole fraction, such as at 0.0001 mole fraction, and preferably at 0 mole fraction. Zinc oxide may compromise hepatic function if it is present in a sufficiently high concentration and if it is capable of leaching out of the glass material. It is desirable to keep zinc oxide below 0.01 mole fraction, such as at 0.001 or 0.0001 mole fraction, and preferably at 0 mole fraction. It is desirable to keep iron oxide below 0.01 mole fraction, such as at 0.001 or 0.0001 mole fraction, and preferably at 0 mole fraction.

The authors of the present disclosure have identified glass compositions with characteristics that are particularly desirable for vascular embolization applications. The glass compositions are shown in Table 1.

The compositions for the "TRCR#" and "BIC#" formulations are reported based on the relative portions of the starting material; however, the actual compositions of the produced glass may differ slightly from the theoretical composition.

The compositions for the "BIC# - IGP" formulations are reported based on measured values of the corresponding irregular glass powders sieved to retrieve particulates ≤45 μm. These compositions are believed to more accurately reflect the actual composition of the produced glass.

The compositions for the "BIC# - MPS" formulations are reported based on measured values of substantially spherical microparticulate glass material produced from the corresponding BIC# - IGP composition. The glass microspheres were sieved to obtain microspheres with a mean size range between 20 μm to 30 μm. The composition of the irregular glass particles changes when the particles are flame-treated to re-melt the surface of the irregular particles and subsequently allowed to form the substantially spherical droplets.

The compositions for the "BIC# - MPC" formulations are reported based on measured values of post-conditioned material produced from the corresponding BIC# - MPS. The substantially spherical microparticulate glass material was post-conditioned with calcium- and magnesium-free phosphate buffered saline (CMF-PBS) at 0.2 g/mL and 50 °C for 72 hours to 366 hours. The composition of the flame-treated particles changes when the particles are conditioned to reduce surface reaction deposits arising as a result of the flame-treatment.

The compositions for the "BIC2 - MPC#" formulations are reported based on measured values of substantially spherical post-conditioned microparticulate glass material produced from the irregular BIC2 glass powders, where the different compositions for the formulations are a result of different flame treatment parameters. The irregular BIC2 glass powders were sieved to retrieve particulates ≤45 μm, flame-treated to re-melt the surface of the irregular particles and allowed to form the substantially spherical droplets, sieved to obtain microspheres with a mean size range between 20 μm to 30 μm, and post-conditioned with calcium- and magnesium-free phosphate buffered saline (CMF-PBS) at 0.2 g/mL and 80 °C for 72 hours.

Although the composition of a glass may differ when comparing (a) the portions of the starting reagents to (b) the measured composition of the produced glass to (c) substantially spherical conditioned microparticles useful for embolic vascularization, it should be understood that in the present disclosure they all refer to the same vascular embolization product at various stages of production. Accordingly, for example, the BIC2 composition that is "about 0.69 mole fraction of $SiO_2$" based on the theoretical mol% of components also refers to (i) the irregular glass powder having "about 0.66 mole fraction of $SiO_2$", (ii) the post-spherodized glass material having "about 0.73 mole fraction of $SiO_2$", and (iii) the post-conditioned glass materials having "about 0.74 mole fraction $SiO_2$", "about 0.77 mole fraction $SiO_2$", "about 0.79 mole fraction $SiO_2$" or "about 0.80 mole fraction $SiO_2$". Unless specifically noted, the mole percentages/fractions discussed herein refer to the theoretical percentages/fractions based on the portions of the starting reagents.

Table 1 - Exemplary glass compositions of the present disclosure

| Formulation | SiO$_2$ in mol.% (wt.%) | Y$_2$O$_3$ in mol.% (wt.%) | BaO in mol.% (wt.%) | Na$_2$O in mol.% (wt.%) | B$_2$O$_3$ in mol.% (wt.%) | Ta$_2$O$_5$ in mol.% (wt.%) |
|---|---|---|---|---|---|---|
| TRCR3 | 71.6289 (46) | 5.02989 (12) | - | 17.0049 (11) | - | 6.33633 (30) |
| TRCR8 | 64.3464 (44) | 10.8840 (28) | 7.76911 (14) | 7.32122 (5) | 9.2029 (7) | 0.476408 (2) |
| TRCR9 | 55.0000 (36) | 7.22562 (18) | 20.0000 (34) | 12.0526 (8) | 5.72178 (4) | - |
| TRCR12 | 60.1593 (32) | 11.1523 (22) | 5.37755 (7) | 15.3458 (8) | - | 7.96503 (31) |
| TRCR13 | 59.3141 (39) | 0.000 (0) | 12.1752 (21) | 9.78519 (7) | 14.0132 (11) | 4.71235 (23) |
| TRCR16 | 55.0000 (32) | 1.12500 (2) | 9.51973 (14) | 20.0000 (12) | 5.70004 (4) | 8.65523 (36) |
| TRCR20 | 67.4457 (54) | 0.000 (0) | 15.7327 (32) | 16.8216 (14) | - | - |
| TRCR23 | 61.9275 (32) | 1.11526 (2) | 20.0000 (27) | 7.85729 (4) | - | 9.1000 (35) |
| BIC1 | 70.827 (45) | 6.239 (15) | - | 16.652 (11) | - | 6.282 (45) |
| BIC1 - IGP | 72.720 (46.60) | 6.112 (14.72) | - | 15.203 (10.05) | - | 5.966 (28.12) |
| BIC1 - MPS | 76.134 (49.10) | 6.320 (15.32) | - | 11.784 (7.84) | - | 5.672 (27.33) |
| BIC2 | 68.716 (44) | - | 6.806 (11) | 16.284 (11) | 1.187 (1) | 7.007 (44) |
| BIC2 - IGP | 65.925 (45.90) | - | 6.511 (11.57) | 20.327 (14.60) | 1.066 (0.86) | 6.171 (31.60) |
| BIC2 - MPS | 73.288 (45.90) | - | 7.301 (11.67) | 10.881 (7.03) | 1.130 (0.82) | 7.400 (34.09) |
| BIC2 - MPC | 73.879 (44.50) | - | 8.457 (13.00) | 8.626 (5.36) | 0.573 (0.04) | 8.465 (37.50) |
| BIC2 - MPC2 | 78.5 (49.3) | - | 7.6 (12.14) | 6.3 (4.06) | 0.30 (0.22) | 7.4 (34.25) |
| BIC2 - MPC3 | 77.0 (43.7) | - | 10.0 (14.5) | 3.1 (1.8) | 0.16 (0.11) | 9.6 (39.8) |
| BIC2 - MPC4 | 79.7 (47.7) | - | 8.2 (12.6) | 3.3 (2.1) | 0.13 (0.94) | 8.5 (37.5) |
| BIC3 | 58.215 (37) | 2.438 (6) | 3.502 (6) | 20.000 (13) | 9.203 (7) | 6.642 (37) |
| BIC3 - IGP | 65.417 (41.50) | 2.470 (5.89) | 3.650 (5.91) | 16.732 (10.95) | 4.924 (3.62) | 6.807 (31.76) |
| BIC3 - MPS | 73.006 (42.40) | 3.129 (6.83) | 4.588 (6.80) | 8.946 (5.36) | 1.664 (1.12) | 8.666 (37.02) |

The density of the irregular microparticulate glass material sieved to retrieve particulates ≤45 $\mu$m, the density of the substantially spherical microparticulate glass material, and the radiopacity of the substantially spherical glass compositions are shown in Table 2.

Table 2 - Radiopacity and densities for exemplary glass compositions of Table 1

| Formulation | Irregular material (IGP) (g/cm$^3$) | substantially spherical material (MPS) (g/cm$^3$) | CT Radiopacity (HU) at 70 kVp | CT Radiopacity (HU) at 120 kVp |
|---|---|---|---|---|
| TRCR3 | 3.3869±0.0033 | 3.3742±0.0021 | 11,453±390 | 13,042±1,681 |
| TRCR8 | 3.3166±0.0010 | 3.3570±0.0048 | 16,152±381 | 9,978±579 |
| TRCR9 | 3.5940±0.0011 | 3.6451±0.0045 | 18,340±1,021 | 13,268±1,004 |
| TRCR12 | 4.0227±0.0015 | 4.0524±0.0029 | 17,448±917 | 19,138±1,170 |
| TRCR13 | 3.4828±0.0050 | 3.5239±0.0047 | 17,821±967 | 16,381±520 |

(continued)

| Formulation | Irregular material (IGP) (g/cm$^3$) | substantially spherical material (MPS) (g/cm$^3$) | CT Radiopacity (HU) at 70 kVp | CT Radiopacity (HU) at 120 kVp |
|---|---|---|---|---|
| TRCR16 | 3.9072±0.0016 | 3.7679±0.0041 | 19,119±1,362 | 20,827±1,427 |
| TRCR20 | 3.0505±0.0038 | 2.7526±0.0030 | 16,305±918 | 9,992±550 |
| TRCR23 | 4.2545±0.0018 | 4.2674±0.0045 | 18,867±1,180 | 21,882±1,160 |
| BIC1 | 3.4967±0.0059 | 3.5000±0.0032 | 13,113±531 | 13,676±642 |
| BIC2 | 3.4589±0.0040 | 3.4326±0.0057 | 15,873±821 | 15,964±650 |
| BIC3 | 3.5415±0.0054 | 3.5739±0.0026 | 16,393±185 | 16,952±205 |

The glass used in TheraSphere™ yttrium-90 microspheres, which includes 40 wt.% $Y_2O_3$, 20 wt.% $Al_2O_3$, and 40 wt.% $SiO_2$ (referred to herein as "YAS4" or "COMP1")was used as a control in various tests, as disclosed in greater detail below. The YAS4 irregular glass powder, sieved to retrieve particulates ≤45 $\mu$m, had a measured composition of 65.795 mol% of $SiO_2$ (36.56 wt%), 19.111 mol% of $Al_2O_3$ (20.90 wt%), and 15.095 mol% of $Y_2O_3$ (42.40 wt%). The substantially spherical microparticulate YAS4 glass had a measured composition of 64.448 mol% of $SiO_2$ (38.12 wt%), 19.492 mol% of $Al_2O_3$ (20.89 wt%), and 16.060 mol% of $Y_2O_3$ (40.70 wt%).

In contrast to the exemplary glass compositions above, the YAS4 glass has: a density of 3.3466±0.0060 g/cm$^3$ for the irregular glass powder sieved to retrieve particulates ≤45 $\mu$m; a density of 3.3078±0.0077 g/cm$^3$ for the substantially spherical material; a CT Radiopacity at 70 kVp of 10,387±245 HU for the substantially spherical material; and a CT Radiopacity at 120 kVp of 5,955±202 HU for the substantially spherical material, as measured using the CT Radiopacity measurement method discussed herein.

The glass formulations TRCR3, TRCR9, TRCR12, TRCR13, TRCR16, TRCR23, BIC1 - MPS, BIC2 - MPS and BIC3 - MPS all show CT Radiopacity of more than 13,000 HU at 120 kVp, which is more than double the radiopacity of the control YAS4 glass composition. Of these formulations, TRCR3, TRCR9, TRCR13, BIC1 - MPS, BIC2 - MPS and BIC3 - MPS have densities for the substantially spherical microparticulate glass material that are within 10% of the density of the control YAS4 glass composition.

**Glass materials.** The substantially spherical microparticles according to the present disclosure, which may be useful for embolic vascularization, are produced by first forming a bulk glass. The bulk glass is then processed to provide irregular microparticulate glass material according to the present disclosure. The irregular microparticles are flame treated to form the substantially spherical microspheres. Flame treatment of irregular glass microparticles to form substantially spherical microspheres is well known in the art. Examples of flame treatment include flame spherodization ultrasonic spray pyrolysis, droplet generator, and vertical thermal flame. Different glass compositions of the present disclosure may melt at different temperatures. Flame treatment processes used to re-melt the surfaces of irregular microparticles may use different gases or gas mixtures, such as a propane-oxygen or acetylene-oxygen, in order to provide a temperature that can re-melt the surface of an irregular glass microparticle of interest. Microparticles that are spherodized by flame-treatment may be conditioned to reduce or remove surface reaction deposits arising as a result of the flame-treatment.

In the context of the present disclosure, the terms "microparticle" and "microparticulate" may be used interchangeably, and refer to a particle that has a diameter that is less than 1200 $\mu$m. For a mixture of particles, the mixture has an average diameter that is less than 1200 $\mu$m.

Although the present disclosure may refer to "microspheres" or "glass microspheres" or "spherical particles", it should be understood that particles of the present disclosure do not need to be perfectly spherical. In the context of the present disclosure, "substantially spherical" refers to a mixture of particles that have an average percent sphericity ("% SPHT") of at least 90%. The percentage sphericity (% SPHT) may be determined using a CamSizer P4 (ATS Scientific, Burlington, ON) system, operating on dynamic image analysis principle, per ISO ISO9276-6 and ISO133322-2, respectively.

% SPHT can be determined using the following equation:

$$\% \, \mathrm{SPHT} = \frac{4\sqrt{A}}{P^2} \times 100$$

where P is the measured perimeter/circumference of a particle projection and A is the measured area covered by a particle projection; such that an ideal microsphere %SPHT is expected to be as 100%.

The terms "microsphere" and "substantially spherical microparticle" may be used interchangeably and refer to a substantially spherical particle that has an average diameter that is less than 1200 $\mu$m. Mixtures of microspheres have an average diameter that is less than 1200 $\mu$m.

Bulk glasses according to the present disclosure may have a glass composition as discussed above, which may reflect the theoretical noted mol% of components. One specific example of such a bulk glass is a glass of BIC2, which has a theoretical composition of 0.687 mole fraction of $SiO_2$; 0.163 mole fraction of $Na_2O$; 0.068 mole fraction of BaO; about 0.070 mole fraction of $Ta_2O_5$; and about 0.012 mole fraction of $B_2O_3$.

Irregular microparticles may be produced by pulverizing the bulk glass using any technique well known in the art, for example by using a planetary ball mill comprising $ZrO_2$ grinding media, and sieving the resulting particles to retrieve particulates of a desired size. Using $ZrO_2$ as a grinding media may help reduce process contaminants due to the toughness of the grinding media relative to the bulk glass. One specific example of irregular microparticles according to the present disclosure is a glass of BIC2 - IGP, which has a composition of 0.659 mole fraction of $SiO_2$; 0.146 mole fraction of $Na_2O$; 0.065 mole fraction of BaO; about 0.062 mole fraction of $Ta_2O_5$; and about 0.011 mole fraction of $B_2O_3$. Other specific examples are the glasses of BIC1 - IGP and BIC3 - IGP.

The irregular microparticles may have an average diameter from about 15 $\mu$m to about 1200 $\mu$m. Difference sized microparticles may be used in different vascular embolization protocols. The microparticles of the present disclosure may be selected to preferentially distribute in tumour vasculature over normal tissue. The size of the microparticles affects this distribution. Microparticles according to the present disclosure, for example that are useful for producing microspheres for visualizing or treating liver tumours, may have average diameters from about 15 $\mu$m to about 45 $\mu$m. In particular examples, the microparticles may have average diameters from about 20 $\mu$m to about 35 $\mu$m. In other examples, irregular microparticles of the present disclosure may be sieved to provide particles from about 40 $\mu$m to about 500 $\mu$m; from about 40 $\mu$m to about 300 $\mu$m; from about 300 $\mu$m to about 500 $\mu$m; from about 500 $\mu$m to about 700 $\mu$m; or from about 700 $\mu$m to about 1200 $\mu$m. Microspheres obtained from the different sizes of microparticles may be selected depending on the internal diameters of the blood vessels to be occluded. For example, blood vessels that are further from a solid tumour but that still provide blood to support tumour growth may be larger in diameter than blood vessels found within the tumour. It may be desirable to use larger particles to block the larger blood vessels, even if the microspheres are not useful for visualizing the tumour itself.

It should be understood that "about X $\mu$m" in the context of particle size and particle diameter is determined based on accepted tolerances as per ASTM E-11 for a test sieve of the noted size. For example, the accepted tolerance for a 50 $\mu$m test sieve is 3 $\mu$m. Accordingly, "about 50 $\mu$m" refers to particles that are from 47 $\mu$m to 53 $\mu$m in size. In another example, the accepted tolerance for a 35 $\mu$m test sieve is 2.6 $\mu$m. Accordingly, "about 35 $\mu$m" refers to particles that are from 32.4 $\mu$m to 38.6 $\mu$m in size. The ASTM accepted tolerance for a 25 $\mu$m sieve is 2.2 $\mu$m. For test sieves without a standard, accepted tolerance (such as test sieves below 20 $\mu$m), the expression "about X $\mu$m" refers to $\pm$15% for sizes from 5 to 15 $\mu$m, and $\pm$50% for sizes less than 5 $\mu$m. For example "about 1 $\mu$m" refers to particles that are from 0.5 to 1.5 $\mu$m in size.

Irregular microparticles may be flame-treated to re-melt their surfaces and allowing a substantially spherical particle to form. Flame-treating irregular microparticles may be achieved with flame spherodization by introducing appropriately sized irregular microparticles into a propane/oxygen flame, and directing the flame into a collection system.

One specific example of a substantially spherical microparticle according to the present disclosure is a glass of BIC2 - MPS, which has a glass composition of 0.733 mole fraction of $SiO_2$; 0.109 mole fraction of $Na_2O$; 0.073 mole fraction of BaO; 0.074 mole fraction of $Ta_2O_5$; and 0.011 mole fraction of $B_2O_3$. Other specific examples are the glasses of BIC1 - MPS and BIC3 - MPS.

Conditioning glass microspheres to reduce surface reactivity is well known in the art. The conditioning may be achieved, for example, by exposing flame-treated microparticles, preferably with agitation at 120 rpm, to a CMF-PBS solution for at least 72 hours at a temperature of 80 °C, followed by rinsing with ultra high purity water. The microparticles may be at a concentration of 0.2 g of microparticles per mL of CMF-PBS. One specific example of a conditioned microparticle according to the present disclosure is a glass of BIC2 - MPC, which has a glass composition of 0.739 mole fraction of $SiO_2$; 0.086 mole fraction of $Na_2O$; 0.085 mole fraction of BaO; 0.085 mole fraction of $Ta_2O_5$; and 0.006 mole fraction of $B_2O_3$.

Spherodizing irregular microparticles is not expected to substantially change the diameter of the particles. However, the spherodized particles may be sieved, either before or after conditioning, to provide particles of the desired size.

**Imaging.** Radiopaque glass microspheres according to the present disclosure may be used for X-ray based imaging, such as radiography imaging, computerized tomography (CT) imaging, cone beam CT imaging, or fluoroscopy imaging.

A desirable radiopacity of the glass microspheres may depend on the clinical scenario, such as the type of imaging being used, the target treatment area, and/or the estimated packing density of the microspheres. A higher radiopacity glass would be desirable when a relatively small number of microparticles are being delivered, the microparticles are expected to be distributed across a relatively large area, a relatively low-power imaging technique is used, or any combination thereof. Conversely, since too high a radiopacity has the potential to result in imaging artifacts and a decrease in imaging quality, a lower radiopacity glass would be desirably when a relatively larger number of microparticles are being delivered, the microparticles are expected to be distributed across a relatively small area, a relatively high-power imaging technique is used, or any combination thereof.

Glass microspheres according to the present disclosure that are sized to be below 45 $\mu$m and that have a density from 2.7 g/cm$^3$ to about 4.3 g/cm$^3$, such as from about 2.9 g/cm$^3$ to about 3.7 g/cm$^3$, or from about 3.3 g/cm$^3$ to about 3.6 g/cm$^3$, may be useful in applications where the microspheres are administered via intra-arterial or intravenous delivery. Glass microspheres according to the present disclosure having a density of about 3.3 g/cm$^3$ would have substantially the same density as a currently available commercial radioactive microsphere.

Some glass microspheres according to the present disclosure may be compatible with positron emission tomography (PET), single-photon emission computed tomography (SPECT), and/or magnetic resonance imaging (MRI) in that the glass microspheres do not affect the PET, SPECT or MRI imaging.

For imaging applications where the glass microspheres are administered via intra-arterial or intravenous delivery to a patient in order to image the patient's liver, at least about 750 microspheres per gram of liver may be administered to the patient. In some examples, about 1000 to about 5000 microspheres per gram of liver may be administered. For a typical adult human patient, about 1 million to about 7 million microspheres may be administered.

**Radiotherapeutic mixtures, compositions, delivery devices, and methods.** Radioactive microparticles are manufactured only in a small number of locations, and prepared for delivery to hospitals around the world. The specific activity of the microparticles are calibrated to provide a desired activity at the planned time of administration. For example, TheraSphere, a yttrium-90 glass microparticle, are prepared by neutron activation of yttrium-89 containing glass microparticles to produce microparticles having a nominal specific activity of about 110 GBq/g at the time of calibration, and are typically provided in amounts of about 1.2 million microparticles (about 3 GBq in about 27 mg) to 8 million microparticles (about 20 GBq in about 180 mg) per vial. Depending on the delay between calibration and administration, the amount of activity available to be delivered per vial may range from 0.17 GBq (1.2 million microparticles injected 9 days after calibration) to 18 GBq (8 million microparticles injected 1 day after calibration).

As mentioned above, for a given amount of delivered radioactivity, it is believed that administering more microparticles with a lower specific activity is desirable because increasing the number of microparticles results in better tumour coverage in comparison to administering fewer microparticles at a higher specific activity. For example, in order to administer 3 GBq of radioactivity to a patient, it is believed that administering 6 million microparticles with an overall specific activity of 22 GBq/g results in better tumour coverage than administering 1.5 million microparticles at 88 GBq/g.

Without wishing to be bound by theory, the authors of the present disclosure believe that, for at least some tumor sizes and/or degrees of vascularization, administering to the patient a mixture of (i) radioactive microparticles; and (ii) non-radioactive microparticles according to the present disclosure may provide at least some of the benefits associated with administering more microparticles at a lower specific activity, even if the individual radioactive microparticles are at a higher specific activity.

The present disclosure provides a mixture of (i) radioactive glass microparticles; and (ii) non-radioactive, radiopaque microparticulate glass material according to the present disclosure. The radioactive glass microparticles are suitable to treat tumors in the liver. The radioactive glass microparticles and the non-radioactive radiopaque microparticulate glass material have substantially the same size. Particles that are substantially the same size are expected to behave in substantially the same way after injection into a patient. Accordingly, administering a mixture of particles that are substantially the same size is believed to result in a homogeneous distribution of the radioactive and non-radioactive particles.

In the context of the present disclosure, particles having substantially the same size refers to the average sizes of (a) the radioactive microparticles and (b) the radiopaque, non-radioactive microparticles being within 40% of the average of the two average sizes. For example, the radioactive microparticles may have an average diameter of 20 $\mu$m, while the radiopaque, non-radioactive microparticles may have an average diameter of 30 $\mu$m. The difference of 10 $\mu$m between the two types of microparticles is 40% of the average of the two values. The smaller the size difference, the more similar the particles are expected to behave. Accordingly, it may be preferable for the difference in average sizes to be within 10% of the average of the two averages sizes.

The density of the microparticles may also affect their behavior after injection into a patient. In particular examples, the radiopaque microparticles of the present disclosure and the radioactive glass microparticles have substantially the same density.

The term "particle density" refers to the weight of an individual particle per unit volume. This is in contrast to the term "bulk density", which refers to the weight of many particles per total volume. Particle density is an intrinsic property of the material, while bulk density will change depending on the properties of the materials in the total volume. Particle density may be discussed in terms of specific gravity, which is the ratio of the density of a substance to the density of a reference substance. In the context of the present disclosure, specific gravity is in reference to water. In the context of the present disclosure, particles having substantially the same density refers to particles that are within about 30%, and preferably within about 15%, of the average.

The particle densities of the (a) radioactive microparticles, and (b) the radiopaque, non-radioactive microparticles may be within about 30%, and preferably within about 15%, of the average. For example, the radioactive microparticles may have a particle density of 3.3 $g/cm^3$, while the radiopaque, non-radioactive microparticles may have a particle density of 3.9 $g/cm^3$. The difference of 0.6 $g/cm^3$ between the two types of microparticles is 16.7% of the average of the two values.

Mixtures according to the present disclosure may be made using any radiopaque glass composition disclosed herein.

The mixture may be prepared by exposing pre-radioactive glass microparticles to neutron activation to form the radioactive glass microparticles, and combining the radioactive glass microparticles with the radiopaque microparticulate glass material of the present disclosure.

In one particular example, the mixture includes (i) substantially spherical radioactive yttrium oxide-aluminosilicate glass microparticles comprising about 40 wt% $SiO_2$, about 20 wt% $Al_2O_3$, and about 40 wt% $Y_2O_3$ (which is equivalent to about 0.170 mole fraction $Y_2O_3$, about 0.189 mole fraction $Al_2O_3$, and about 0.641 mole fraction $SiO_2$); and (ii) substantially spherical, radiopaque microparticulate glass material comprising about 0.741 mole fraction of $SiO_2$; about 0.086 mole fraction of $Na_2O$; about 0.085 mole fraction of $BaO$; about 0.085 mole fraction of $Ta_2O_5$; and about 0.006 mole fraction of $B_2O_3$.

Yttrium-89 may be transformed into yttrium-90 by exposing yttrium-89 containing microparticles to a neutron flux. The specific activity of the resulting microparticles is dependent on the level of flux and the duration of the exposure. For example, yttrium-89 may be exposed to a flux of nominally $10^{14}$ neutrons/$cm^2$/sec to effect neutron activation for a number of days to achieve a specific activity of >150 GBq/g.

An improvement in tumour coverage, for example a more uniform distribution of microparticles, may be achieved with mixtures having radioactive microparticles in an amount from about 80% to about 10% w/w of the total mass of microparticles in the composition. It should be understood that, in the context of the present disclosure, reference to any improvement is in comparison to the same number of radioactive microparticles in the absence of additional non-radioactive microparticles.

With radioactive microparticles having a high specific activity, such as 140 GBq/g, the mixtures may have fewer radioactive microparticles (such as around 10 wt%). In contrast, with radioactive microparticles having a low specific activity, such as 4 GBq/g, the mixtures may have more radioactive microparticles (such as around 80 wt%). In particular examples, such as with radioactive microparticles having a specific activity of about 88 GBq/g, the mixtures may have about 25 wt% radioactive microparticles.

It should be understood that "specific activity" refers to the radioactivity per unit mass of the radioactive microparticles, while "overall specific activity" refers to the radioactivity per unit mass of the mixture of radioactive and non-radioactive microparticles. For example, taking one gram of radioactive microparticles having a specific activity of 10 GBq/g and mixing those microparticles with one gram of non-radioactive microparticles would result in a mixture of microparticles with an overall specific activity of 5 GBq/g.

The mixture of radioactive and non-radioactive particles may be prepared in formulations at a desired radioactivity with different numbers of total microparticles. The total number of microparticles may be selected based on the tumour size and/or degree of vascularization. For example, a formulation having a radioactivity of 10 GBq in 0.5 grams of microparticles may be desirable to treat a tumour with a certain degree of vascularization, while a formulation having a radioactivity of 10 GBq in 1 gram of microparticles may be desirable to treat a more vascularized tumour.

In still another aspect of the present disclosure, radiation is delivered to a mammal by administering a therapeutic amount of the mixture to the mammal when the mixture includes radioactive glass microspheres. Such a method may additionally include imaging the mammal using an X-ray based radiologic imaging technique. Administering to a patient a therapeutic amount of such a mixture of microparticles may allow for the calculation of a delivered dose of radiation to a tissue by non-imageable radioactive microparticles, based on a measured distribution of the non-radioactive, radiopaque microparticles in the tissue.

Without wishing to be bound by theory, the authors of the present disclosure also believe that at least some of the benefits associated with administering a mixture of radioactive and non-radioactive microparticles can be obtained when administering radioactive and non-radioactive microparticles separately.

In some aspects, the present disclosure provides a therapeutic or diagnostic composition comprising a mixture of radioactive microparticles and non-radioactive microparticles, where at least some of the non-radioactive microparticles are composed of the glass material disclosed herein.

The radioactive microparticles and the non-radioactive microparticles may have a difference in particle densities that is within 30%, and preferably within 15%, of the average of the two particle densities.

The radioactive microparticles may have an average diameter from about 10 to about 1200 microns, such as an average diameter from about 20 to about 40 microns. The non-radioactive microparticles may have an average size from about 10 to about 1200 microns, such as an average diameter from about 20 to about 40 microns. The radioactive microparticles and the non-radioactive microparticles may have a difference in average sizes that is within 40% of the average of the two averages sizes.

In some examples, the radioactive microparticles and the non-radioactive microparticles have substantially the same resistance when flowing in a liquid through a conduit.

A skilled person would understand that the resistance of an object flowing in a liquid through a conduit is reflected by the drag coefficient, and that the drag coefficient is a function of skin friction and form drag. Accordingly, resistance of a microparticle flowing in a liquid through a conduit may be affected by, for example: the size, surface area, shape, density of the microparticle, and/or surface condition of the microparticle. A skilled person would also readily understand that two different particles may have substantially the same resistance flowing in a liquid through a conduit since changing a feature to increase drag may be offset by changing another feature to decrease drag. For example, two particles may still have substantially the same drag coefficient, even though the first particle is larger than the second particle, if the surface condition of the first particle is sufficiently smoother than the surface condition of the second particle.

In the context of the current disclosure, the time it takes for a bolus of microparticles to fall a set distance through a liquid may represent the resistance of the microparticles flowing in a liquid through a conduit. This time may be measured by loading a known number of microparticles into a transparent column filled with distilled water. The number of microparticles should be selected so that the height of the bolus of microparticles is from two to five times the inner diameter of the column. Once the microparticles have settled at the bottom of the column, the column is inverted and the microparticles fall through the distilled water, with the drag counteracting the gravitational force. The total time it takes for the microparticles to fall past a transition point is measured. The transition point, measured from the top of the bolus of microparticles, is at least 100 times the inner diameter of the column. For example, in a column with an inner diameter of 0.5 cm, the settled microparticles may be 1.5 cm high, and the total fall time for the bolus of microparticles is the time it takes for all of the microparticles to fall past a point that is 50 cm away from the top of the settled microparticles.

This total fall time is compared to the total fall time for a substantially equal number of a different group of microparticles tested under the same conditions (i.e. the same fluid, the same column, the same transition point). The relative drag ratio is calculated by dividing the fall time for the first group of microparticles by the fall time for the second group of microparticles. In the context of the present disclosure, the first and the second microparticles would be considered to have substantially the same resistance when flowing in a liquid through a conduit if the relative drag ratio was from about 0.95 : 1 to about 1 : 0.95.

In some examples, the radioactive microparticles make up from about 10% to about 80%, such as about 25%, of the total mass of microparticles in the composition.

In some examples, the radioactive microparticles are diagnostic radioactive microparticles. In some examples, the radioactive microparticles are therapeutic radioactive microparticles.

Diagnostic radioactive microparticles may include one or more radioisotopes selected from the group consisting of: copper-67, holmium-166, indium-111, iodine-131, lutetium-177, molybdenum-99, phosphorus-32, rubidium-82, technicium-99m, and thallium-201.

Therapeutic radioactive microparticles may include one or more radioisotopes selected from the group consisting of: actinium-225, bismuth-213, copper-67, indium-111, iodine-131, iodine-125, gadolinium-157, holmium-166, lead-212, lutetium-177, palladium-103, phosphorus-32, radium-223, rhenium-186, rhenium-188, samarium-153, strontium-89, and tungsten-188.

The radioactive glass microparticles may be substantially spherical. The non-radioactive microparticles may be substantially spherical.

In another aspect, the present disclosure provides a method that includes administering a mixture of radioactive microparticles and non-radioactive microparticles, as described above, to a patient; where the administration is: by intravascular delivery, intra-peritoneal delivery, or percutaneous delivery.

In a further aspect, the present disclosure provides a delivery device for intravascular delivery, intra-peritoneal delivery, or percutaneous delivery of a mixture of radioactive microparticles and non-radioactive microparticles to a patient. The delivery device is fluidly coupleable to a mixing and transport medium, and includes: a fluid inlet fluidly coupleable to the mixing and transport medium; a fluid outlet; a fluid mixer fluidly coupled to the fluid inlet and to the fluid outlet; a source of radioactive microparticles fluidly coupled to the fluid mixer; and a source of non-radioactive microparticles fluidly coupled to the fluid mixer. The source of the radioactive microparticles is distinct from the source of non-radioactive microparticles. The fluid mixer mixes radioactive microparticles with the non-radioactive microparticles, and delivers the mixture of radioactive and non-radioactive microparticles out of the fluid outlet utilizing the mixing and transport medium. At least some of the non-radioactive microparticles are composed of a glass material according to the present disclosure.

In still another aspect, the present disclosure provides a delivery device for intravascular delivery, intra-peritoneal delivery, or percutaneous delivery of a mixture of radioactive microparticles and non-radioactive microparticles to a patient. The delivery device includes: at least one fluid inlet fluidly coupleable to a transport medium; a source of radioactive microparticles fluidly coupled to the at least one fluid inlet; a source of non-radioactive microparticles fluidly coupled to the at least one fluid inlet; a first fluid outlet fluidly coupled to the source of the radioactive microparticles; and a second fluid outlet fluidly coupled to the source of non-radioactive microparticles. The source of the radioactive microparticles is distinct from the source of non-radioactive microparticles. At least some of the non-radioactive microparticles are composed of a glass material according to the present disclosure. In some examples, the delivery device delivers the radioactive microparticles and the non-radioactive microparticles in a single treatment session. In some examples, the first fluid outlet and the second fluid outlet are proximate to each other. In the context of the present disclosure, it should be understood that the fluid outlets are proximate to each other if the patient could be administered the radioactive microparticles and the non-radioactive microparticles at substantially the same time, for example over the course of a single treatment session.

The radioactive microparticles in the delivery devices may be any radioactive microparticle disclosed herein. The non-radioactive microparticles in the delivery devices may be any non-radioactive microparticle disclosed herein. In some examples, the radioactive microparticles make up from about 10% to about 80%, such as about 25%, of the total mass of microparticles in the delivery device.

In a still further aspect, the present disclosure provides a method that includes mixing (i) a first population of radioactive microparticles and (ii) a second population of non-radioactive microparticles, and administering a therapeutically or diagnostically relevant amount of the mixture to a patient. At least some of the non-radioactive microparticles are composed of a glass material according to the present disclosure. The radioactive microparticles used in the method may be any radioactive microparticle disclosed herein. The non-radioactive microparticles used in the method may be any non-radioactive microparticle disclosed herein. In some examples, the radioactive microparticles make up from about 10% to about 80%, such as about 25%, of the total mass of microparticles used in the method. The administration may be by intravascular delivery, intra-peritoneal delivery, or percutaneous delivery.

In other aspects, the present disclosure provides a method of administering a therapeutically or diagnostically relevant amount of microparticles to a patient. The method includes either: administering non-radioactive microparticles to the patient, and administering radioactive microparticles to the patient without first detecting the non-radioactive microparticles; or administering radioactive microparticles to the patient, and administering non-radioactive microparticles to the patient without first detecting the radioactive microparticles. At least some of the non-radioactive microparticles are composed of a glass material according to the present disclosure. The administration is by intravascular delivery, intra-peritoneal delivery, or percutaneous delivery. The route of administration of the non-radioactive microparticles is the same as the route of administration of the radioactive microparticles.

In some examples, the method includes concurrent administration of the non-radioactive and the radioactive microparticles. In other examples, the method includes sequential administration of the non-radioactive and the radioactive microparticles; or sequential administration of the radioactive and the non-radioactive microparticles.

In still another aspect, the present disclosure provides a method of administering a therapeutically or diagnostically relevant amount of microparticles. The method includes: concurrent administration of (i) a first population of radioactive microparticles and (ii) a second population of non-radioactive microparticles to a patient. At least some of the non-radioactive microparticles are composed of a glass material according to the present disclosure.

In some examples, the first population of radioactive microparticles is distinct from the second population of non-radioactive microparticles. The first population of radioactive microparticles and the second population of non-radioactive microparticles may be administered as a mixture.

In yet another aspect, the present disclosure provides a method of administering a therapeutically or diagnostically relevant amount of microparticles. The method includes sequential administration in a single treatment session of non-radioactive microparticles, and of radioactive microparticles to a patient. At least some of the non-radioactive microparticles are composed of a glass material according to the present disclosure.

In still another aspect, the present disclosure provides a method that includes sequential administration to a patient of (i) therapeutically radioactive microparticles, and then (ii) non-radioactive microparticles. At least some of the non-radioactive microparticles are composed of a glass material according to the present disclosure.

In some examples, sequential administration includes intermittent administration of the non-radioactive microparticles and the radioactive microparticles. The intermittent administration may include alternating administration of the non-radioactive microparticles and the radioactive microparticles.

In some examples, sequential administration includes administration of all of one type of microparticles before administration of the next type of microparticles. For example, sequential administration may include administration of all of the non-radioactive microparticles before administration of any of the radioactive microparticles; or administration of all of the radioactive microparticles before administration of any of the non-radioactive microparticles.

Methods according to the present disclosure may deliver a therapeutically relevant amount of radiation to the patient, or may deliver a diagnostically relevant amount of non-radioactive microparticles to the patient.

In methods according to the present disclosure: the administration may be by intravascular delivery, intra-peritoneal delivery, or percutaneous delivery; the radioactive microparticles and/or the non-radioactive microparticles may be as discussed above; about 10% to about 80%, such as about 25%, of the total mass of microparticles delivered may be radioactive microparticles; or any combination thereof.

Although the above discussion relates to methods of administering radioactive microparticles and non-radioactive microparticles, the present disclosure equally contemplates the corresponding "uses" of the microparticles, including microparticles useful in the disclosed methods, and uses of microparticles in the manufacture of an administrable formulation useful in the disclosed methods.

In the context of the present disclosure, it should be understood that the non-radioactive microparticles discussed in this section of the disclosure may have any of the features, alone or in combination, of the glass materials discussed in the section above entitled "Glass materials". For example, the non-radioactive microparticles may have any or all of the features associated with microparticles, microspheres, glass microspheres, or spherical particles discussed above.

In the context of the present disclosure, it should be understood that the radioactive microparticles discussed in this section of the disclosure may have any of the features, alone or in combination, of the radioactive glass materials discussed in this disclosure.

**Examples**

**Irregular Glass Powder and Microsphere Synthesis.** Various glasses according to the present disclosure were synthesized. The theoretical compositions of the TRCR# and BIC# glasses are shown in Table 1, above. In addition, comparative glasses COMP# were also synthesized. The theoretical compositions of those comparative examples are shown in Table 3.

Table 3 - Compositions of comparative glasses

| Formulation | $SiO_2$ in mol.% (wt.%) | $Y_2O_3$ in mol.% (wt.%) | BaO in mol.% (wt.%) | $Na_2O$ in mol.% (wt.%) | $B_2O_3$ in mol.% (wt.%) | $Ta_2O_5$ in mol.% (wt.%) | $Al_2O_3$ in mol.% (wt%) |
|---|---|---|---|---|---|---|---|
| COMP1 | 64.074 (40) | 17.048 (40) | - | - | - | - | 18.878 (20) |
| COMP2 | 64.328 (37) | 15.671 (34) | 20.000 (29) | - | - | - | - |
| COMP3 | 74.237 (65) | - | 7.683 (17) | 11.5718 (11) | 6.3675 (6) | - | - |

The density of the irregular microparticulate glass material sieved to retrieve particulates ≤45 $\mu$m, the density of the substantially spherical microparticulate glass material, and the radiopacity of the substantially spherical glass compositions are shown in Table 4.

Table 4 - Radiopacity and densities for comparative glass compositions of Table 3, where "insufficient mass" denotes that the composition was not tested due to inability to obtain the minimum mass required to conduct the test

| Formulation | Irregular material (g/cm³) | Substantially spherical material (g/cm³) | CT Radiopacity (HU) at 70 kVp | CT Radiopacity (HU) at 120 kVp |
|---|---|---|---|---|
| COMP1 | 3.3466±0.0060 | 3.3078±0.0077 | 10,387±245 | 5,955±202 |
| COMP2 | 3.8950±0.0032 | insufficient mass | insufficient mass | insufficient mass |
| COMP3 | 2.6827±0.0069 | 2.3606±0.0038 | 10,627±261 | 5,633±169 |

The comparative example "COMP1" corresponds to YAS4 disclosed in United States Patent 4,789,501. Due to the clinical utility of this glass, and the known composition-structure-property relationships, the authors of the present disclosure used this comparative example to identify glass compositions with substantially equivalent microsphere densities (±10%), enhanced radiopacity, acceptable in vitro cytotoxicity, acceptable hemocompatibility, and/or acceptable genotoxicity. Without wishing to be bound by theory, the authors of the present disclosure expect that glass compositions that meet many or all of these requirements would be useful for application in transarterial radioembolization.

For glass synthesis, analytical grade reagents were weighted in accordance with the theoretical compositions outlined in Table 1 and Table 3. The reagents were homogeneously blended for ≥1 hour prior to subsequent placement in platinum-rhodium crucibles (50 cc to 200 cc); fired (1500 °C to 1600 °C, 3 hours to 5 hours) using an electric furnace (Carbolite Furnaces, Sheffield, UK) and then shock quenched into water for injection (USP, Ph. Eur. Grade, Rocky Mountain Biologics, MT, US). The obtained irregular glass powder was dried in an oven (120°C) overnight.

All of the exemplary glasses of the present disclosure, as well as COMP1 and COMP3, easily melted to provide non-viscous fluids prior to the shock quenching. The glass of COMP2 did not melt, phase separated, or provided a viscous fluid.

Combined irregular glass powder from various melts were pulverized as a lot using a planetary ball mill comprising $ZrO_2$ grinding media. The resulting ground irregular glass powder was sieved to retrieve particulates $\leq 45\ \mu m$.

Appropriately classified irregular glass powder was then introduced into a propane/oxygen flame where the flow of oxygen and propane were appropriately controlled. The materials were re-melted, and spherical liquid droplets were formed by surface tension, in a process otherwise known as spherodization. The flame of the burner was directed into a stainless-steel collection system which collected the glass microspheres as they were expelled from the flame. The glass microspheres were subsequently sieved, to obtain microspheres with a mean size range between 20 $\mu m$ to 30 $\mu m$.

**X-ray diffraction.** X-ray diffraction (XRD) measurements for irregular glass powders and/or microspheres was performed using a Bruker D2 Phaser diffractometer (Bruker AXS Inc., Madison, WI) coupled to an X-ray generator (30 kV; 10 mA) and equipped with a Cu target X-ray tube. Specimens of each experimental material were prepared by pressing the irregular glass powders or microspheres into hollow zero-background holders. Powder diffraction powders were acquired in the scan angle range $10°<2\theta<100°$ with a step size of $0.02°$. Collection time for the XRD spectra was 4930 s.

**Helium Pycnometry.** The density for irregular glass powders and/or microspheres was measured using a helium pycnometer (AccuPyc II 1340, Micromeritics), with the average of ten to sixty measurements ($\pm$ standard deviation, SD) recorded for the materials post quench in irregular glass powder form and an average of ten measurements ($\pm$SD) in final microsphere form, respectively.

**Differential Scanning Calorimetry.** The thermal characteristics for irregular glass powders was characterized by a Netzsch Pegasus F404 differential scanning calorimeter (DSC, Burlington, MA). DSC measurement were conducted at a heating rate of 10 °C/minute from ambient temperature to 1000 °C, to graphically determine the glass transition temperature ($T_g$, onset) at onset and midpoint ($T_g$, midpoint) using the Proteus 6.0 software. The tolerance of the DSC used in this work is 2%.

**Microsphere Conditioning.** To obtain substantially smooth surface morphologies that are substantially free of any surface reaction deposits arising as a result of spherodization, the flame-treated microspheres were subjected to a conditioning process post spherodization, through extraction in calcium and magnesium free phosphate buffered solution (CMF-PBS, Product Code: MT21040CV, Corning™, NY, US) at a ratio of 0.2 g/mL.

For the initial study, the microspheres were extracted within an enclosed container in a shaking water bath, at 50 °C for $72 \pm 2$ hours, $120 \pm 2$ hours, $240 \pm 2$ hours, $288 \pm 2$ hours, and $360 \pm 2$ hours, under continuous agitation at 120 rpm. Subsequent to this, further analysis was completed for microspheres extracted at an accelerated condition (80 °C) within an enclosed container in a shaking water bath for $24 \pm 2$ hours and $72 \pm 2$ hours, under continuous agitation at 120 rpm to investigate the potential to facilitate a conditioning step designed for manufacturing.

Post extraction, the microspheres were separated from CMF-PBS and rinsed (10 times) with sterile water for injection (USP, Ph. Eur. Grade, Rocky Mountain Biologics, MT, US) prior to drying at $120 \pm 2$ °C until a constant mass (difference in weight $\leq 0.1\%$) was obtained.

The glass microspheres were subsequently stored for analysis or re-sieved, and size sorted to ensure microspheres with a final mean size of 20 $\mu m$ to 30 $\mu m$ prior to packaging in cleaned glass storage vials for bulk storage.

Microsphere cleanliness (i.e. substantially free from surface reaction deposits and/or contaminants by visual inspection) were subsequently verified by scanning electron microscopy (SEM) analysis. Microsphere extracts were obtained during the conditioning study noted above at $72 \pm 2$ hours, $120 \pm 2$ hours, $240 \pm 2$ hours, $288 \pm 2$ hours, and $360 \pm 2$ hours were analyzed for Si, Na, Ta, B and Ba elemental concentrations by ICP-OES at an ISO17025 certified laboratory (NSL Analytical, 4450 Cranwood Pkwy, Warrensville Heights, OH, US) using validated test protocols.

**Compositional Analysis.** For compositional analysis, irregular glass powders and/or microspheres were subjected to sample preparation by fusion/microwave acid digestion and analyzed by ICP-OES at an ISO17025 certified laboratory (NSL Analytical, 4450 Cranwood Pkwy, Warrensville Heights, OH, US) using validated test protocols.

**Scanning Electron Microscope.** Carbon coated morphologies for irregular glass powder and/or microspheres were examined using a Hitachi S-4700 field emission SEM (FE-SEM), operating at an accelerating voltage of 1.5KV to 10.0KV accelerating voltage, about 15 $\mu A$ to 22 $\mu A$ emission current, and about 12 mm to 14 mm working distance. SEM images were subsequently obtained at 250X and 1000X magnifications.

**Axial Computed Tomography (CT) Scanning and Assessment of MRI Safety.** Bulk microsphere CT radiopacity was assessed through quantitative radiopacity measurements, expressed as Hounsfield Unit Values (HU) obtained from five replicate regions of interest (ROIs, n=5) recorded from respective Axial CT scans (1 mm slice thickness, pitch=0.5, 70 kVp and 120 kVp) through 1.2 mL glass v-vials (Product Code: Z115061, Sigma Aldrich, Canada) with 500 mg microspheres in 6 $\mu L$ of sterile saline. All measurements were performed on the experimental materials within mean ($\pm$SD) diameter of 20 $\mu m$ to 30 $\mu m$, using a Siemens Somatom Definition AS+ scanner (Siemens Healthcare, Erlangen, Germany) and the extended HU range option employed for scanning.

To study the effect of microsphere concentration on quantitative CT radiopacity and MRI safety, five separate concentrations of BIC1 to BIC3 microspheres versus the COMP1 control were prepared in the following volume fractions (in triplicate): 1.25%, 2.5%, 5%, 7.5% and 10%. A gel only tube (0%) was also used as a baseline control. All volume fractions were calculated using the microsphere densities per Table 2 and Table 4 and prepared in 1.5 mL of 8% gelatin; within 5 mm NMR tubes vigorously shaken and rolled once gelatin was added to microspheres to optimize the homogeneity of the microsphere distribution prior to quickly setting on ice. If the tubes were overly heterogeneous, the gel melted, and the microspheres were redistributed, and subsequently reset using ice. Due to microsphere density, some clustering of microspheres around the periphery of the tube and at the bottom formed.

CT data was acquired at 80 kVp on a Trifoil preclinical PET/CT scanner, at a sample resolution of 100 $\mu$m with all CT images converted to HU for subsequent analysis. MRI data was acquired on a 3T Varian MRI system with an INOVA console. For bulk T1, T2 and T2* quantitative measurements, a birdcage RF transmit/receive coil was used, whereby the bulk measurements represented the portion of the tube that was in the coil and not restricted to a single slice, with tubes positioned such that the bulk of the microspheres were within the imaging region of the coil. Qualitative MRI measurements were also acquired using a standard gradient-echo sequence (T2*-weighted), a spin-echo sequence (T2-weighted), and a short TR anatomical localizer scan (T1-weighted). Note that for improved scientific comparisons, all quantitative parameters are written as R1, R2 or R2*, where the relaxivity (R) is inversely related to the time decay constant (T), where R1 is 1/T1, R2 is 1/T2, and R2* is 1/T2*.

Five imaging characteristics were thus evaluated: CT radiopacity, R1 relaxivity, R2 relaxivity, R2* relaxivity and Susceptibility. For each characteristic, the R1 (or R2, etc.), for example, of each tube was measured and compared to a control gel tube resulting in a calculated Delta (D) R1. DR1 was then graphed vs volume fraction for each set of tubes, and a linear regression line was plotted.

**In Vitro Cytotoxicity.** The biological reactivity of a mammalian cell monolayer, L929 mouse fibroblast, in response to the experimental materials was determined in accordance with ISO10993-5:2009, Biological Evaluation of Medical Devices - Part 5: Tests for *In Vitro* Cytotoxicity using certified biocompatibility test laboratories (Toxikon Corporation, Bedford, MA, and Nelson Labs, Salt Lake City, UT and Nelson Labs LLC, Salt Lake City, UT, US).

The experimental materials were prepared using a mass-to-volume extraction ratio of 0.2 g/mL in accordance with ISO10993-12: 2012, Biological Evaluation of Medical Devices - Part 12: Sample Preparation and Reference Materials. Positive (Natural Rubber) and negative (Plastic) control articles were prepared to verify test system functionality.

The experimental and control material extracts were used to replace the maintenance medium of the cell culture. The experimental material extract was tested at 100% (NEAT), 50%, 25% and 12.5% serial dilutions in serum-supplemented (complete) Minimum Essential Medium (MEM) with or without 5% bovine serum (1XMEM5).

All cultures were incubated in at least 6 replicates for 24±2 hours (for pre-conditioned microspheres during initial screening) or 72±2 hours (for post-conditioned microspheres to facilitate microsphere optimization) at 37 ± 1 °C, in a humidified atmosphere containing 5±1 % $CO_2$.

The viability of cells following the exposure to the extracts was measured via their capacity to uptake a vital dye, MTT reagent. This dye was added to the cells to be actively incorporated in viable cells. The number of viable cells correlates to the color intensity determined by photometric measurements at 570 nm after extraction.

**In Vitro Genotoxicity.** This study was conducted to satisfy, in part, the requirements of the ISO10993-3: 2014, Biological evaluation of medical devices - Part 3: Tests for genotoxicity, carcinogenicity and reproductive toxicity and the Organisation for Economic Cooperation and Development (OECD) 471, and Guideline for Testing of Chemicals: Bacterial Reverse Mutation Test using a certified biocompatibility test laboratory (NAMSA Corporation, Northwood, OH, US).

The test article, control, and experimental blank (extraction vehicle without the test article) were prepared using a mass-to-volume extraction ratio of 0.2 g/mL in accordance with ISO10993-12: 2012, Biological Evaluation of Medical Devices - Part 12: Sample Preparation and Reference Materials in both DMSO and Saline at 50 °C for 72 hours under continuous agitation. Following extraction, all extracts were visually inspected for colour, turbidity and particulate and were maintained at room temperature for less than 3 hours before use. The extracts were not centrifuged, filtered, or otherwise altered. Prior to dosing and testing, tester strain cultures (S. typhimurium tester strain TA98. TA100. TA1535 and TA1537, and E. coli tester strain WP2uvrA) were checked for genetic markers.

Following this, molten top agar was supplemented with histidine-biotin solution or tryptophan solution for the S. typhimurium or E. coli tester strains, respectively. This addition allowed the bacteria on the plate to undergo several divisions to produce a faint background lawn, visible to the naked eye, which could be examined under a darkfield colony counter. Separate tubes containing 2.0 mL of supplemented molten top agar were inoculated with 0.1 mL of culture for each of the five tester strains, and 0.1 mL of the DMSO or saline test article extract. A 0.5 mL aliquot of sterile water for injection or S9 homogenate, providing metabolic activation, was added when necessary. The mixture was poured across triplicate Minimal E plates labeled with lab number, appropriate tester strain, and S9 metabolic activation (when applicable). Parallel testing was also conducted with each negative control and six positive controls. Histidine-free media plates (for S. typhimurium) and tryptophan-free media plates (for E. coli) were prepared in triplicate as follows:

    **I.** DMSO and saline test article extracts in the presence and absence of S9 activation

II. Negative controls in the presence and absence of S9 activation

III. Benzo[a]pyrene in the presence of S9 and 2-nitrofluorene in the absence of S9 activation with strain TA98

IV. 2-Aminoanthracene in the presence of S9 and sodium azide in the absence of S9 activation with strain TA100

V. 2-Aminoanthracene in the presence of S9 and sodium azide in the absence of S9 activation with strain TA1535

VI. 2-Aminoanthracene in the presence of S9 and ICR-191 in the absence of S9 activation with strain TA1537

VII. 2-Aminoanthracene in the presence of S9 and MMS in the absence of S9 activation with strain WP2uwA

The plates were incubated at 37 °C for 2 days. Following the incubation period, the revertant colonies on each plate were recorded, with mean number of revertants and standard deviations determined. The mean number of revertants on the test plates were then compared to the mean number of revertants on the negative control plates for each of the tester strains employed, with the background lawn characteristics also recorded.

**In vitro Hemocompatibility.** The procedure uses the principles outlined in ASTM F756: 2017. The ASTM method has been validated using human blood which is in compliance with ISO 10993-4: 2017, Biological evaluation of medical devices - Part 3: Selection of tests for interactions with blood using a certified biocompatibility test laboratory (Nelson Labs, Salt Lake City, UT and Nelson Labs LLC, Salt Lake City, UT) in compliance with US FDA good manufacturing practice (GMP) regulations 21 CFR Parts 210, 211 and 820, without deviation from procedures. Testing followed the principles outlined in ASTM F756: 2017, for which calcium and magnesium free PBS was used as the extraction solvent and the ASTM method validated using human blood, citrated, in compliance with ISO 10993-4: 2017 which states due to differences in blood activity, human blood should be used where possible.

For blood collection, an equal amount of blood from 3 donors was drawn into vacutainers containing 0.1 M sodium citrate at a ratio of 9:1 (3.2% anticoagulant to blood). The collected blood was refrigerated until testing was performed, with blood pooled and used for testing within four hours of draw. A hemoglobin standard was diluted with Drabkin's reagent to give solutions at concentrations of 0.80, 0.60, 0.40, 0.30, 0.20, 0.10, 0.02, and 0.01 mg/mL. The solutions were allowed to stand at room temperature for a minimum of 15 minutes and the absorbance read on a spectrophotometer at 540 nanometers (nm). A standard curve was subsequently determined using linear regression with the absorbance values and the standard concentrations of hemoglobin. To determine plasma hemoglobin, blood was centrifuged at 700-800 x g for 15 minutes. A 1mL aliquot of the plasma was then added into 1 mL of Drabkin's reagent and placed at room temperature for a minimum of 15 minutes before the absorbance was read on a spectrophotometer at 540 nm. The hemoglobin concentration was determined from the standard curve and then multiplied by a factor of 2 to obtain the plasma free hemoglobin concentration (confirmed as less than 2 mg/mL). The total amount of hemoglobin present was determined by adding a 20 $\mu$L aliquot of the blood to 5 mL of Drabkin's reagent, in duplicate, and allowing the solution to stand at room temperature for a minimum of 15 minutes prior to reading the absorbance on a spectrophotometer at 540 nm. The hemoglobin concentration was then determined from the standard curve and multiplied by a factor of 251 to account for the dilution. Based on the total hemoglobin present, the blood was diluted to 10 $\pm$ 1 mg/mL in CMF-PBS. To verify the blood dilution, a 300 $\mu$L aliquot of the diluted blood was added to 4.5 mL of Drabkin's reagent in triplicate and allowed to stand at room temperature for a minimum of 15 minutes before reading the absorbance on a spectrophotometer at 540 nm. The hemoglobin concentration was subsequently determined from the standard curve and multiplied by a factor of 16 to account for the dilution.

The experimental materials positive controls (hemolytic), negative controls (non-hemolytic) and the blank were prepared using a mass-to-volume extraction ratio of 0.2 g/mL or surface area to volume extraction ratio of 6 cm$^2$/mL in accordance with ISO10993-12: 2012, Biological Evaluation of Medical Devices - Part 12: Sample Preparation and Reference Materials, with samples for indirect contact (extract) analysis prepared in CMF-PBS at 50 $\pm$ 2 °C (with agitation) for 72 $\pm$ 2 hours. For both the indirect (extract) and direct contact methods, appropriate concentrations of the extracts or materials were added to labelled glass tubes, prepared in triplicate, and 1 mL of diluted blood added to each. The glass tubes were then incubated at 37 $\pm$ 2 °C for a minimum of 3 hours, with gentle inversions twice at 30-minute intervals throughout the incubation period. After incubation, the test articles and controls were centrifuged at 700-800 x g for 15 minutes and 1 mL of the supernatant fluid was combined with 1 mL of Drabkin's reagent and allowed to stand at room temperature for a minimum of 15 minutes. Following the centrifugation, the supernatant of the test articles, blank and controls were visually inspected for colour, turbidity and particulate. Subsequent to this, all test articles and controls were then read at 540 nm in a spectrophotometer.

The Hemolytic index (% Hemolysis) was interpreted using the following equation:

$$\text{Hemolytic Index} = \frac{\text{Hemoglobin Released (mg/mL)}}{\text{Hemoglobin Present (mg/mL)}} \times 100$$

where: Hemoglobin Released (mg/mL) = (Optical Density x X Coefficient + Constant) x 16

Hemoglobin Present (mg/mL) = Diluted Blood 10 ± 1 mg/mL

The corrected hemolytic index was calculated by subtracting the hemolytic index of the CMF-PBS blank solution from the hemolytic index of the test article and controls, with the test article compared to the negative control by subtracting the hemolytic index of the negative control from the hemolytic index of the test article.

**Statistical Analysis.** CT and MRI measurements, expressed as the mean (±Standard Error Mean, SEM) of three replicates, were subjected to linear regression analyses for each microsphere formulation prepared in various volume fractions was statistically evaluated to ensure it was non-zero and furthermore compared to other formulations using Prism 8.0 software (GraphPad. Software Inc. San Diego, CA, US). The level of significance set at $p < 0.05$.

**Amorphous Morphology, Density and Glass Transition Temperature.** Exemplary glasses of the present disclosure, and comparative glasses, were assessed for amorphous morphology, density and glass transition temperature in the form of irregular glass powders and microspheres. All samples remained amorphous, irrespective of subsequent heat treatment (spherodization) post irregular glass powder making, such that no identifiable crystalline peaks were detected in any of the formulations assessed. Table 5 presents the density, and $T_g$ (midpoint, $T_gM$, and at inflection, $T_gI$), for the comparative glasses and the exemplary glasses of the present disclosure in both irregular glass powder and microsphere form post-spherodization.

Table 5 - Density and glass transition temperatures for exemplary and comparative glasses, , where "insufficient mass" denotes that the composition was not tested due to inability to obtain the minimum mass required to conduct the test

| Formulation | Irregular glass powder: Density (g/cm$^3$) | Microsphere: Density (g/cm$^3$) | Irregular glass powder: $T_g$, M (°C) | Irregular glass powder: $T_g$, I(°C) |
|---|---|---|---|---|
| **COMP1** | 3.3466±0.0060 | 3.3078±0.0077 | 909 | 915 |
| COMP2 | 3.8950±0.0032 | Insufficient mass | 902 | 908 |
| COMP3 | 2.6827±0.0069 | 2.3606±0.0038 | 572 | 580 |
| **TRCR3** | 3.3869±0.0033 | 3.3742±0.0021 | 784 | 787 |
| **TRCR8** | 3.3166±0.0010 | 3.3570±0.0048 | 748 | 752 |
| **TRCR9** | 3.5940±0.0011 | 3.6451±0.0045 | 657 | 659 |
| **TRCR12** | 4.0227±0.0015 | 4.0524±0.0029 | 794 | 799 |
| **TRCR13** | 3.4828±0.0050 | 3.5239±0.0047 | 667 | 680 |
| **TRCR16** | 3.9072±0.0016 | 3.7679±0.0041 | 686 | 664 |
| **TRCR20** | 3.0505±0.0038 | 2.7526±0.0030 | 512 | 513 |
| **TRCR23** | 4.2545±0.0018 | 4.2674±0.0045 | 782 | 776 |
| **BIC1** | 3.4967±0.0059 | 3.5000±0.0032 | 773 | 776 |
| **BIC2** | 3.4589±0.0040 | 3.4326±0.0057 | 663 | 657 |
| **BIC3** | 3.5415±0.0054 | 3.5739±0.0026 | 697 | 701 |

For density, the commercial control "COMP1", provided a density of approximately 3.3 g/cm$^3$, with only minor variation between the irregular glass powder to microsphere form. In summary, the TRCR glass series provides for a broad range of densities from as low as ca. 3.05 g/cm$^3$ (TRCR20) to as high as 4.2674 g/cm$^3$ (TRCR23), similarly with no appreciable difference noted for each of the formulations between irregular glass powder to microsphere form. On review of the TRCR formulations themselves, an increased trend in frit and microsphere densities was observed with incremental $Ta_2O_5$ in the glass network. The BIC formulations exhibited similar trends in density between irregular glass powders to microsphere form (3.459 g/cm$^3$ to 3.542 g/cm$^3$ and 3.4326 g/cm$^3$ to 3.5739 g/cm$^3$, respectively).

For glass transition temperature analysis, the commercial control comprised of $T_gM$ and $T_gI$ data-points between 909°C ($T_gM$) and 915°C ($T_gI$). By comparison, the TRCR and BIC glass series exhibited a broad range of $T_gM$ and $T_gI$ data-points between 512 °C to 794 °C and 513 °C to 799 °C, respectively.

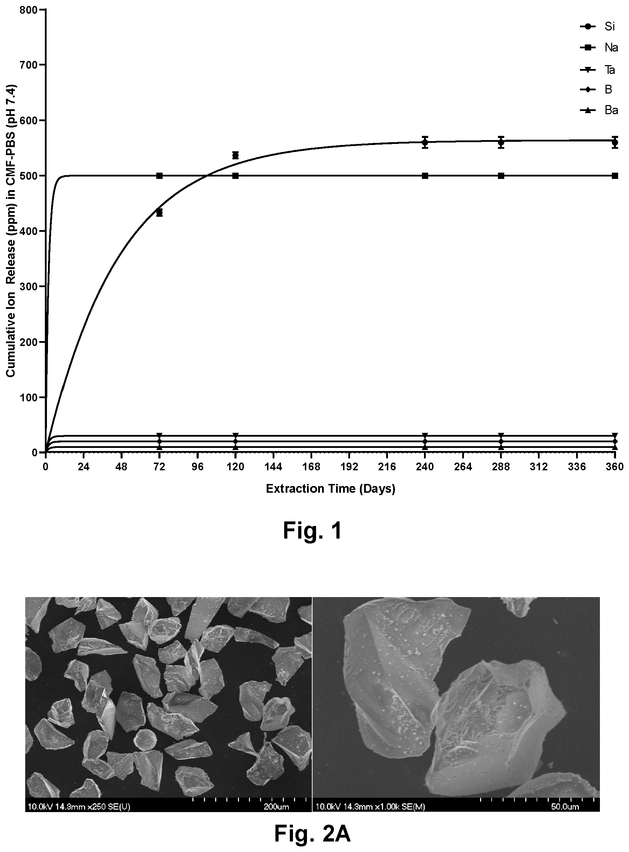

**Conditioning Study.** Fig. 1 illustrates the cumulative ion release curve obtained for BIC2 microspheres (post-spherodization) subjected to extraction in CMF-PBS at 0.2 g/mL up to 360 hours (when maintained at 50 °C under continuous agitation (120 rpm)). In summary, Ba was the only element recorded below the ICP-OES detection limit (10 ppm) after a 72-hour extraction period, with B, Ta, Na and Si detected at 20 ppm, 30 ppm, 500 ppm and between 430 ppm to 440 ppm, respectively. An extraction timeframe of 120 hours subsequently confirmed exhaustive extraction (reported as less than the ICP-OES detection limit (10 ppm)) for all remaining elements, with the exception Si (530 ppm to 540 ppm), which was subsequently shown to reach exhaustive extraction between 240 hours to 360 hours.

**Chemical Analysis and Surface Morphology.** Table 6 shows the variation in chemical composition for the COMP1 article versus BIC formulations used as feed material (irregular glass powders) for spherodization versus the collected microspheres post-spherodization (processing the same lot per formulation).

In summary, all three BIC formulations were observed to undergo significant volatilization of alkali elements post-spherodization, as observed by the significant reduction in $Na_2O$ concentration (in terms of wt.% as reported by the external test laboratory) by up to half the feed material's starting composition (10.05 wt.% to 7.84 wt.% (BIC1); 1460 wt.% to 7.03 wt.% (BIC2) and 10.95 wt.% to 5.36 wt.% (BIC3)). Congruently, other elemental concentrations (i.e. $Ta_2O_5$ and/or $SiO_2$) were observed to compensate for this loss in relativity, by an apparent increase of their elemental concentrations post-spherodization. Further to this, BIC3 demonstrated an apparent volatilization of $B_2O_3$ through spherodization as result of the significant reduction in $B_2O_3$ concentration (3.62wt.% to 1.12wt.%). By comparison, the COMP1 article (TABLE) when used as feed material remained compositionally unchanged (i.e. within $\pm 3$ wt.% (acceptable) variance noted between each elemental constituent).

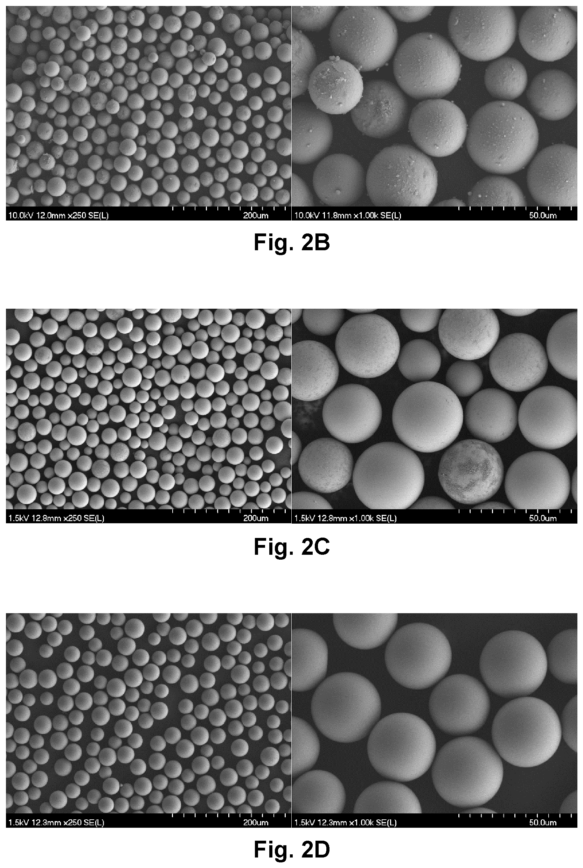

The surface morphology of the feed material is shown in Fig. 2A in comparison to the microsphere surface morphologies post-spherodization (Fig. 2B). The collected SEM images for the as-spherodized materials demonstrates collection of surface reaction deposits on the microspheres that may be attributed to: (I) an accumulation of surface deposits caused by glass reaction with the gaseous environment; and/or (II) contamination with debris from the glass making process or particulate matter in the air or on product contact surfaces.

Table 7 shows the variation in chemical composition as a result of conditioning a separate BIC2 microsphere lot after 360 hours; previously shown in Fig. 1 to have reached exhaustive extraction of $Na_2O$ from the glass network as result of the aqueous environment. The reduction of $Na_2O$ as a result of this process step by comparison to spherodization is less extensive (7.03 wt.% to 5.36 wt.% versus 14.60 wt.% to 7.03 wt.%, respectively), and maybe predominantly attributed to the conditioning step removing the non-bound surface reaction deposits prior to the initial extraction timeframe assessed (72 hours), with minimal dissolution for Na2O from the glass network thereafter.

Leveraging the findings of this study, further extraction analyses (for process optimization) were undertaken at an accelerated extraction temperature and reduced timeframe of 24 hours (with all other variables, such as, extraction ratio, temperature and agitation rate remaining unchanged) to identify whether microspheres free of surface reaction deposits could be observed by SEM. Fig. 2C illustrates microspheres conditioned for 24 hours at 80 °C provide for near complete removal of surface reaction deposits and/or contaminants observed immediately post-spherodization (Fig. 2B) versus microspheres conditioned for 72 hours at 80 °C and re-sieved post-spherodization, that clearly demonstrate complete removal of surface reaction deposits (Fig. 2D). The conditioned BIC2 microspheres after 24 hours and 72 hours were subsequently stored for biocompatibility assessment.

Table 6 Compositional analysis of irregular glass powders versus microspheres (post-spherodization) reported in mol. % to the nearest one thousandth decimal place. (wt.% is shown in parentheses to the nearest one hundredth decimal place)

| Component in mol.% (wt.%) | COMP1 | | BIC1 | | BIC2 | | BIC3 | |
|---|---|---|---|---|---|---|---|---|
| | IGP | MPS | IGP | MPS | IGP | MPS | IGP | MPS |
| $SiO_2$ | 65.795 (36.56) | 64.448 (38.12) | 72.720 (46.60) | 76.134 (49.10) | 65.925 (45.90) | 73.288 (45.90) | 65.417 (41.50) | 73.006 (42.40) |
| $Al_2O3$ | 19.111 (20.90) | 19.492 (20.89) | - | - | - | - | - | - |
| $Y_2O3$ | 15.095 (42.40) | 16.060 (40.70) | 6.112 (14.72) | 6.320 (15.32) | - | - | 2.470 (5.89) | 3.129 (6.83) |
| BaO | - | - | - | - | 6.511 (11.57) | 7.301 (11.67) | 3.650 (5.91) | 4.588 (6.80) |
| $Na_2O$ | - | - | 15.203 (10.05) | 11.784 (7.84) | 20.327 (14.60) | 10.881 (7.03) | 16.732 (10.95) | 8.946 (5.36) |
| $B_2O_3$ | - | - | - | - | 1.066 (0.86) | 1.130 (0.82) | 4.924 (3.62) | 1.664 (1.12) |

(continued)

| Component in mol.% (wt.%) | COMP1 | | BIC1 | | BIC2 | | BIC3 | |
|---|---|---|---|---|---|---|---|---|
| | IGP | MPS | IGP | MPS | IGP | MPS | IGP | MPS |
| $Ta_2O_5$ | - | - | 5.966 (28.12) | 5.672 (27.33) | 6.171 (31.60) | 7.400 (34.09) | 6.807 (31.76) | 8.666 (37.02) |

Table 7 Compositional analysis of BIC2 glass microspheres (pre and post-conditioning) reported in mol. % to the nearest one thousandth decimal place. (wt.% is shown in parentheses to the nearest one hundredth decimal place)

| Component in mol.% (wt.%) | MPC (0 Hour Microsphere Extraction) | MPC (366 Hour Microsphere Extraction) |
|---|---|---|
| $SiO_2$ | 71.890 (44.10) | 73.879 (44.50) |
| BaO | 8.250 (12.90) | 8.457 (13.00) |
| $Na_2O$ | 11.123 (7.03) | 8.626 (5.36) |
| $B_2O_3$ | 0.592 (0.42) | 0.573 (0.40) |
| $Ta_2O_5$ | 8.055 (36.30) | 8.465 (37.50) |

**CT Radiopacity.** The exemplary glass compositions of Table 1 providing enhanced radiopacity in comparison to the control article (COMP1: $5,952\pm180$ HU) at a clinically relevant tube potential (120 kVp). Benchtop CT radiopacity was observed to range from $9,978\pm579$ HU (TRCR8) to $21,858\pm1,037$ HU (TRCR23). The densities of TRCR 3, 8 and 13 were with 10% of the density of the comparative article COMP1, and their radiopacities were observed to range between $9,978\pm579$ HU (TRCR8) to $16,374\pm465$ HU (TRCR13).

BIC1, BIC2, and BIC3, having densities within a desirable target range that is comparable to the control article COMP1, exhibited radiopacity ranging $13,676\pm642$ HU to $16,952\pm205$ HU.

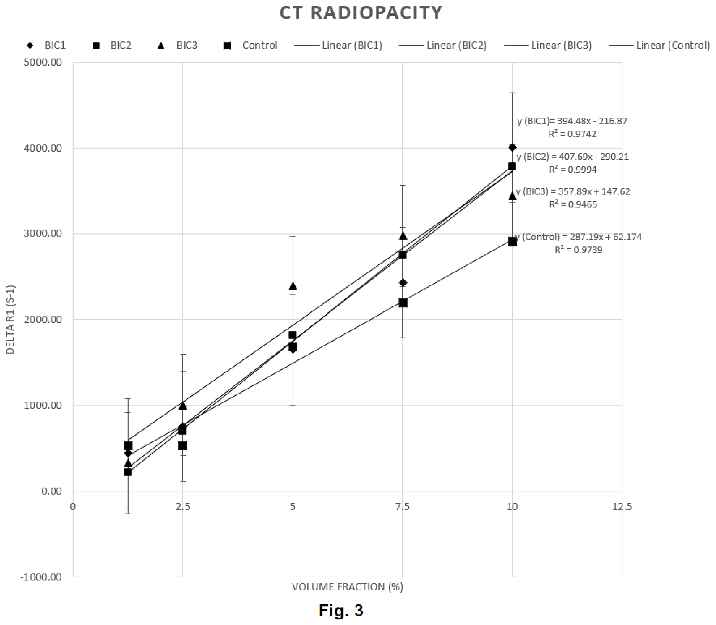

For assessment of CT Radiopacity of variable BIC1 to BIC3 microsphere concentrations versus the control article, Fig. 3 illustrates increasing radiopacity with incremental microsphere concentrations (volume fraction) of the prepared samples comprising variable volume fractions at 80 kVp. Furthermore, good correlation exists between the HU values obtained and the microsphere volume fractions, whereby the slope and $R^2$ value for each of the calibration curves developed were as follows (from low to high CT radiopacity): COMP1 (287.2 at $R^2 = 0.9739$); BIC3 (357.9 at $R^2 = 0.9465$); BIC1 (394.5 at $R^2 = 0.9994$) and BIC2 (407.7 at $R^2 = 0.9742$). The data supports BIC1 to BIC3 as promising candidate materials to offer enhanced radiopacity in comparison to the COMP1 article at microsphere concentrations expected to be administered for therapeutic purposes.

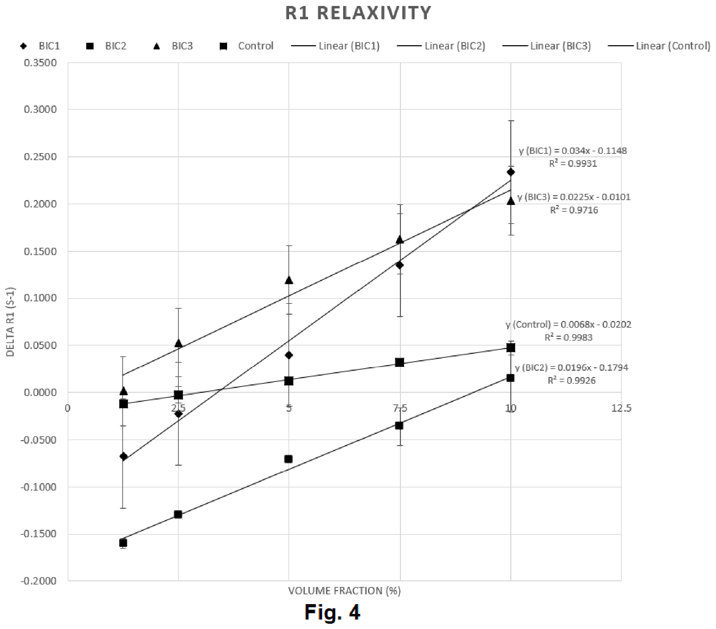

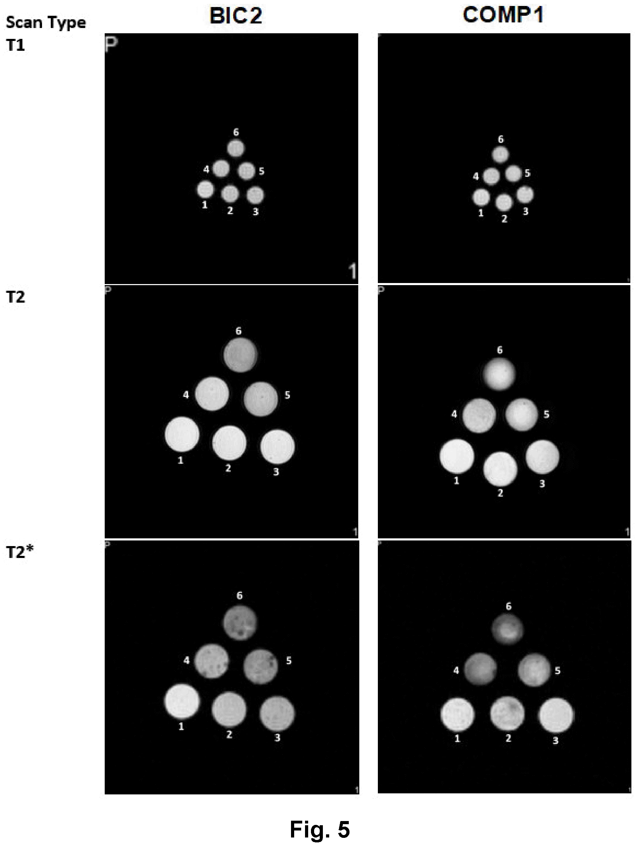

**MRI Assessment.** The BIC1 to BIC3 microsphere formulations and the COMP1 article are not expected to result in large T1 contrast effects, as supported by the calibration curves shown in Fig. 4, developed for R1 relaxivity. The calibration curves show significant correlation between R1 relaxivity and microsphere concentrations. Although the slopes, ranging from 0.007 (COMP1) to 0.034 (BIC3) are statistically non-zero ($p<0.002$), the slope is so small that these microsphere formulations are not expected to cause detectable T1 differences. This is also supported by Fig. 5, where no T1 contrast changes are evident in the images of the tubes.

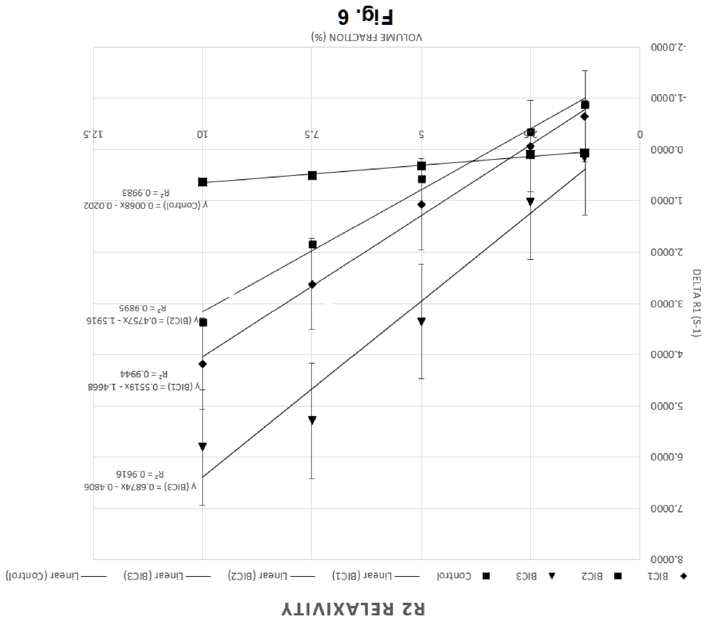

Similarly, BIC1 to BIC3 microsphere formulations or the COMP1 article are not expected to result in large T2 contrast effects, as supported by the calibration curves shown in Fig. 6, developed for R2 relaxivity. The calibration curves show significant correlation between R2 relaxivity and microsphere concentrations. Although the slopes, ranging from 0.068 (COMP1) to 0.688 (BIC3) are statistically non-zero ($p<0.003$), the slopes are sufficiently small that these formulations will not cause large differences in signal contrast compared to physiological differences. This can also be seen in Fig. 5, where there is a very mild T2 contrast change in the highest BIC2 microsphere concentration images.

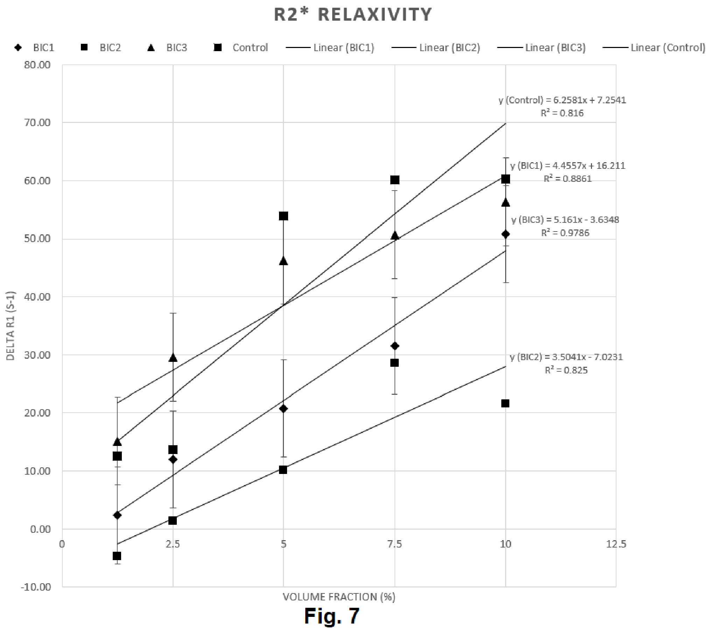

For R2* relaxivity measurements, the calibration curves are shown in Fig. 7. The microsphere formulations will induce some changes in T2* contrast (as shown in Fig. 5). However, given the target location of the microspheres in the vasculature (where there is significant T2* effects due to blood), it is unlikely that the contrast changes will be sufficient to induce significant changes in clinical T2* weighted scans. Similar to the T1 and T2 calibration curves developed, the slopes for R2* relaxivity are statistically non-zero ($p<0.04$), with slopes being in the range of 3.5 s-1 to 6.3 s-1. As shown in Fig. 5, there is a perceptible difference in visible T2* contrast in the highest microsphere concentration for the control article and less so for the BIC2 microspheres, where some bubbles are visible.

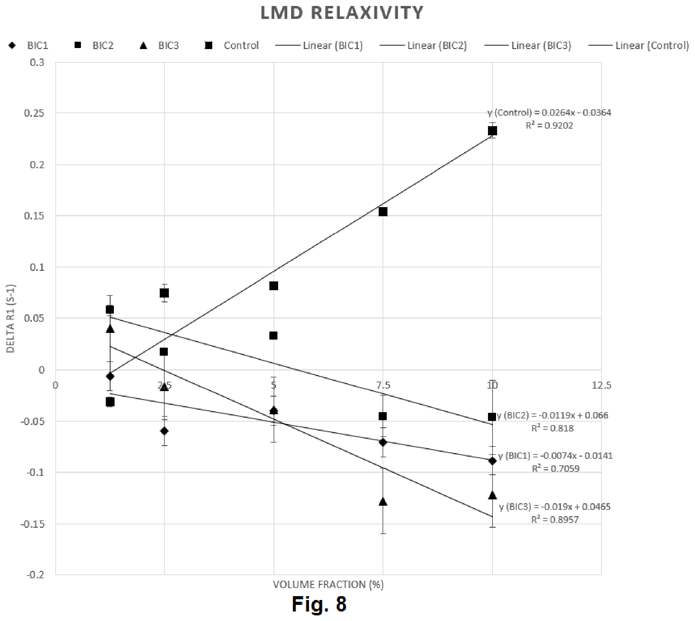

The local magnetic dose (LMD) obtained by the MRI susceptometry results (illustrated in Fig. 8) indicate that BIC1 to BIC3 microsphere formulations are similar with a small negative slope (-0.0074 to -0.0190), indicating that the materials are slightly diamagnetic. This is in contrast to the COMP1 article, which produced a small positive slope (0.0264) to classify it as slightly paramagnetic. BIC1 was not observed as statistically significant from zero (i.e. the susceptibility is neutral), versus BIC2, BIC3 and YAS4 which were all observed as statistically significant from non-zero ($p<0.04$).

To support BIC1, BIC2 and BIC3 microsphere formulations as MRI safe, the following issues were considered: (I) magnetically induced displacement torque, (II) magnetically induced displacement force, and (III) RF induced tissue heating of tissue around the device.

**Magnetically Induced Displacement Torque.** Medical devices may exhibit displacement forces and/or rotational torques resulting from interaction of the magnetic moment of the device, with the main static field of the MRI system. Rotational torque is the tendency for an asymmetrically shaped device that is magnetized along some direction to align the direction of its magnetic moment with the direction of the main magnetic field. Magnetized microspheres, like the BIC and COMP1 microsphere formulations, are symmetrically shaped and have no capacity to produce torque. Torque is calculated from the cross product of the device magnetic moment ($\mu$) and the main static MRI magnetic field ($B_o$). Metal-oxides composed of transition metals, such as the BIC and the COMP1 microsphere formulations, that are in a class of magnetic materials may exhibit either paramagnetism or diamagnetism. These materials have no magnetic moment until placed in an external magnetic field, and the induced magnetic moment will either align (paramagnetism) or perfectly oppose (diamagnetism) the direction of the main static magnetic field. Measurements obtained for suspensions of BIC1 to BIC3 and COMP1, using MRI susceptometry (see Fig. 8), were shown to have a slightly paramagnetic volume susceptibility of 0.0264 ppm (SI units) for the control microspheres themselves (correcting for effects of the suspension media). By comparison, the BIC1, BIC2 and BIC3 microspheres were shown to have slightly diamagnetic volume susceptibility of -0.0074 ppm, -0.0119 ppm and -0.019 ppm, respectively, for the microspheres themselves (correcting for effects of the suspension media).

Since the magnetic moment for diamagnetic materials is in opposition to the main field (by definition), the torque (computed from the cross product of the magnetic moment and main magnetic field, $\tau = \mu \times B$) is zero, and so no risk of particle rotational torque is possible for the BIC1, BIC2 or BIC3 microspheres. Nevertheless, since the magnetic moments are about 0 for all the samples tested, including the control microspheres, none of the microsphere formulations represent a risk of particle rotational torque.

**Magnetically Induced Displacement Force.** Displacement forces create risk of ballistic propulsion of the device into the MRI system (or within a patient's body) and are calculated from the interaction between the device magnetic moment, and the rate of change through space of the main static magnetic field of the MRI system. This spatial main field gradient is typically largest near the entrance to an MRI system (bore entrance) and is on the order of 15 Tesla/M or less for typical 3T clinical MRI systems (e.g. 14.7 T/m for GE Healthcare MR750 3T MRI as specified in safety section of operator manual).

Specifically, displacement forces are calculated from the dot product of the magnetic moment ($\mu$) and the static magnetic field gradient [grad(B)] by the formula [$F = \mu \cdot grad(B)$]. Paramagnetic materials like the BIC2 microspheres develop magnetic moments only as a result of exposure to strong magnetic fields, aligned in the same direction to the applied field. By comparison, diamagnetic materials like the BIC1, BIC2 and BIC3 microspheres develop magnetic moments only as a result of exposure to strong magnetic fields, aligned in the opposite direction to the applied field. Nevertheless, the forces produced for all microsphere formulations are on the order of ppm ($\mu = V^*\Delta\chi$, with V the Radiopaque MS volume) and are small relative to Brownian motion (particle diffusion) forces, and therefore represent no risk to magnetically induced displacement force.

**Radio Frequency (RF) Induced Tissue Heating.** As discussed by Parmar et al. in "Size Dependent Heating Efficiency of Iron Oxide Single Domain Nanoparticles" Procedia Engineering (2015) Volume 102, pages 527-533, RF heating of magnetized particles occurs primarily through magnetic heating mechanisms rather than inductive mechanisms (electrical conduction). Magnetic heating from RF fields may occur either through Neel mechanisms (internal magnetic moment of the particle rotates with applied RF field, without rotating particle) or Brownian mechanisms (particle physically rotates under influence of RF rotating magnetic field creating resistive heating). These mechanisms are relevant for iron-oxide nanoparticles for applications such as magnetic mediated hyperthermia (MMH) and therapeutic applications (targeted cancer ablation). These particles can produce hypothermic conditions (2 °C to 4 °C localized temperature elevation) only under conditions involving RF fields on the order of 10kA/M.

MRI systems conversely produce peak RF fields of approximately 0.1kA/M (1 gauss), or two orders of magnitude weaker than for MMH applications. Additionally, superparamagnetic iron-oxide (SPIO) particles possess magnetic moments approximately 5 orders of magnitude greater than that of the microsphere formulations (0.0264 ppm (COMP1) vs. 0.0074 ppm (BIC1), -0.0119 ppm (BIC2) and -0.019 ppm (BIC3)). Since the BIC1, BIC2 and BIC3 microspheres of the present disclosure are weak paramagnetic / diamagnetic materials, the magnetic heating capability is negligible relative to SPIO agents.

Additionally, SPIO nanoparticles are FDA approved contrast agents (e.g. Ferridex) and produce no issues with RF heating despite being metal-oxide particles, like the BIC1 to BIC3 formulations, even though they have a far greater magnetic moment strength. The capacity for RF heating of the BIC microspheres is several orders of magnitude weaker than for these SPIO agents, due to the weaker magnetic moment, hence they should pose no risk for tissue heating in the MRI environment.

*In Vitro* **Cytotoxicity.** For *in vitro* cytotoxicity screening, the viability of cells exposed to the experimental negative and positive control material extracts were >70% and <70%, respectively, confirming the validity of the assay. Formulations satisfying the requirements of the test demonstrated cell viability percentages of ≥ 70% of the untreated control. Table 8 reports the cell viability results obtained for each pre-conditioned microsphere formulation at variable serial dilutions.

Table 8 - In Vitro Cytotoxicity Results for Pre-Conditioned Microspheres

| Formulation | Cell viability(%) 12.5% Conc. | Cell viability(%) 25% Conc. | Cell viability(%) 50% Conc. | Cell viability (%) NEAT Conc. |
|---|---|---|---|---|
| COMP1 | 97 | 98 | 95 | 94 |
| COMP3 | 95 | 97 | 108 | 109 |
| TRCR3 | 89 | 88 | 93 | 86 |
| TRCR8 | 107 | 106 | 113 | 104 |
| TRCR9 | 98 | 108 | 111 | 112 |
| TRCR12 | 100 | 104 | 94 | 104 |
| TRCR13 | 94 | 85 | 73 | 48 |
| TRCR16 | 93 | 94 | 9 | 106 |
| TRCR20 | 103 | 92 | 87 | 75 |
| TRCR23 | 97 | 92 | 92 | 98 |
| BIC1 | 114 | 120 | 44 | 22 |
| BIC2 | 103 | 100 | 105 | 103 |
| BIC3 | 103 | 100 | 92 | 72 |

The COMP1 article did not exhibit cytotoxic potential, showing cell viabilities between 98 to 94%. In comparison, the majority of the TRCR microsphere formulations were considered not to have a cytotoxic potential, with the exception of TRCR13 tested NEAT (48% cell viability). All other TRCR formulations displayed cell viabilities between 75% to 112% (NEAT, undiluted). With respect to the BIC microsphere formulations, 2 of 3 did not display any cytotoxic potential: namely BIC2 (100% to 105%); and BIC3 (72% to 103%). Cell viabilities of 22% (NEAT, undiluted) and 44% (50% dilution) were noted for BIC1, demonstrating a dose dependent cytotoxic potential.

*In Vitro* **Genotoxicity.** For both the COMP1 article and pre-conditioned BIC 2 microspheres, the background lawn appeared normal for the DMSO and saline test extracts. In no case was there a 2-fold or greater increase in the mean number of revertants for tester strains TA98, TA100 and WP2uvrA or a 3-fold or greater increase in the mean number of revertants for tester strains TA1535 and TA1537 in the presence of DMSO and saline test article extracts. The negative control results for each tester strain exhibited a characteristic number of spontaneous revertants based on historical data collected at the test house. Each positive control mean exhibited at least a 3-fold increase over the respective negative control mean for each of the five tester strains. Table 9 summarizes the results, where the DMSO and saline extracts for both the control article and BIC microsphere formulations were considered to be non-mutagenic to Salmonella typhimurium tester strains TA98, TA100, TA1535, and TA1537, and to Escherichia coli tester strain WP2uvrA.

Table 9 - In Vitro Genotoxicity for Pre-Conditioned Microspheres

| Formulation | *DMSO* | | *Saline* | |
|---|---|---|---|---|
| | **S. Typhimurim** | **E. Coli** | **S. Typhimurim** | **E. Coli** |
| COMP1 | Non-mutagenic | Non-mutagenic | Non-mutagenic | Non-mutagenic |
| BIC1 | Non-mutagenic | Non-mutagenic | Non-mutagenic | Non-mutagenic |
| BIC2 | Non-mutagenic | Non-mutagenic | Non-mutagenic | Non-mutagenic |
| BIC3 | Non-mutagenic | Non-mutagenic | Non-mutagenic | Non-mutagenic |

*In Vitro* **Cytotoxicity and Hemolysis for Post-Conditioned Microspheres.** Table 10 shows the repeat cytotoxicity and/or hemolytic assessment of the BIC2 microspheres conditioned between 24 hours to 72 hours. For *in vitro* cytotoxicity, the validity of the assay was confirmed by media control cells showing normal growth characteristics following the cell exposure step when observed under magnification; positive control extracts (undiluted) reduced the viability to <70% of the media control versus the negative control extracts that did not reduce the viability to <70% of the media control. In summary, the BIC2 microspheres conditioned for 24 hours and 27 hours did not show cytotoxic potential (i.e. a cell viability (CV) of >70%), with cell viabilities ranging between 82% to 84% and 88% to 92%, respectively, when tested at serial dilutions of 12.5% to 50% versus NEAT (undiluted). Subsequently, the conditioning step was verified to have no adverse effect on *in vitro* cytotoxicity, even with prolonged extraction at $72\pm2$ hours prior to MTT analysis compared to the pre-conditioned (0 hours) BIC2 microspheres whose cell viabilities were shown to range between 100% to 105% after a $24\pm2$ hour extraction.

For *in vitro* hemolysis (direct and indirect contact hemolysis), the validity of the assay was confirmed by the difference between the hemolytic indexes of the conditioned BIC2 microspheres and the experimental negative control equals <2% for all testing conditions. Per ASTM F756: 2017, a hemolytic index between 0 to 2 represents a non-hemolytic response and between 2 to 5 represents a slightly hemolytic response versus a response >5 which denotes a hemolytic material response. According to this hemolytic grading system (Table 10):

- Conditioned BIC2 microspheres prepared using the mass to volume extraction ratio (0.2 g/mL) are considered non-hemolytic: 1.29 % to 0.29 % when conditioned between 24 hours to 72 hours, respectively (direct contact method) versus 0.00 % to 0.00 % when conditioned between 24 hours to 360 hours, respectively (indirect contact method).
- Conditioned BIC2 microspheres prepared using the surface-area (SA) to volume extraction ratio (0.2 g/mL) are also considered non-hemolytic: 0.00 % to 0.00 % when conditioned between 24 hours to 72 hours, respectively (direct contact method).

Table 10 - *In Vitro* Cytotoxicity and Hemolysis for Microspheres Pre-Conditioned versus Post-Conditioned from 24 hours to 360 hours. '-' denotes no data available

| BIC2 Microsphere Conditioning Timeframe (Temp.) | CV (%) 12.5% Conc. | CV (%) 25% Conc. | CV (%) 50% Conc. | CV (%) NEAT Conc. | Hemolytic Index: Direct Hemolysis (Mass to Vol.) | Hemolytic Index: Direct Hemolysis (SA to Vol.) | Hemolytic Index: Indirect Hemolysis (Mass to Vol.) |
|---|---|---|---|---|---|---|---|
| *24 h (80 °C)* | 84 | 82 | 82 | 82 | 1.60 | 0.00 | 0.00 |
| *72 h (50/80 °C)* | 92 | 89 | 89 | 88 | 0.29 | 0.00 | 0.00 |
| *120 h (50 °C)* | - | - | - | - | - | - | 0.08 |
| *360 h (50 °C)* | - | - | - | - | - | - | 0.00 |

The data shown in Table 10 confirm that BIC2 microspheres subjected to accelerated conditioning parameters (24 hours at 80 °C) impart no adverse effect on cytotoxicity (82% to 92% post-conditioning versus 100% to 106% post-spherodization) or hemolysis, despite the apparent lack of surface cleanliness observed by SEM per Fig. 2. Nevertheless, a conditioning timeframe of 72 hours was observed to significantly reduce the hemolytic index to 0.29 by the direct contact method.

In the preceding description, for purposes of explanation, numerous details are set forth in order to provide a thorough understanding of the examples. However, it will be apparent to one skilled in the art that these specific details are not required. Accordingly, what has been described is merely illustrative of the application of the described examples and numerous modifications and variations are possible in light of the above teachings.

Particular aspects of the present invention as summarised in the Clauses below:

Clause 1. A glass material, wherein the glass comprises:

from about 0.55 to about 0.85 mole fraction of $SiO_2$;
from about 0.01 to about 0.23 mole fraction of $Na_2O$, $K_2O$, or a combination of $Na_2O$ and $K_2O$;
from about 0.05 to about 0.28 mole fraction of: $Y_2O_3$, BaO, or a combination of $Y_2O_3$ and BaO; and
optionally $Ta_2O_5$,

wherein the sum of the $Y_2O_3$, the BaO and the optional $Ta_2O_5$ is from about 0.10 to about 0.31 mole fraction.

Clause 2. The glass material according to clause 1, wherein the glass comprises from about 0.03 to about 0.23 mole fraction of $Na_2O$, $K_2O$, or a combination of $Na_2O$ and $K_2O$.

Clause 3. The glass material according to clause 2, wherein the glass comprises from about 0.05 to about 0.23 mole fraction of $Na_2O$, $K_2O$, or a combination of $Na_2O$ and $K_2O$.

Clause 4. The glass material according to any one of clauses 1 to 3, wherein the glass comprises from about 0.55 to about 0.82 mole fraction of $SiO_2$.

Clause 5. The glass material according to clause 4, wherein the glass comprises from about 0.55 to about 0.80 mole fraction of $SiO_2$.

Clause 6. The glass material according to clause 1, wherein the glass comprises:

from about 0.55 to about 0.80 mole fraction of $SiO_2$; and
from about 0.05 to about 0.22 mole fraction of $Na_2O$, $K_2O$, or a combination of $Na_2O$ and $K_2O$.

Clause 7. The glass material according to any one of clauses 1 to 6, wherein the sum of the $Y_2O_3$, the BaO and the optional $Ta_2O_5$ is from about 0.10 to about 0.25 mole fraction, such as from about 0.10 to about 0.15 mole fraction.

Clause 8. The glass material according to any one of clauses 1 to 7, wherein the glass comprises from about 0.05 to about 0.12, such as from about 0.05 to about 0.08, mole fraction of $Y_2O_3$, BaO, or a combination of $Y_2O_3$ and BaO.

Clause 9. The glass material according to any one of clauses 1 to 8, wherein the glass comprises $Ta_2O_5$ in an amount up to about 0.12, such as up to about 0.10, mole fraction of $Ta_2O_5$.

Clause 10. The glass material according to clause 9, wherein the glass comprises from about 0.05 to about 0.12, such as from about 0.05 to about 0.10, mole fraction of $Ta_2O_5$.

Clause 11. The glass material according to any one of clauses 1 to 10, wherein the glass further comprises $B_2O_3$ in an amount up to 0.20 mole fraction of $B_2O_3$.

Clause 12. The glass material according to clause 11, wherein the sum of the $B_2O_3$ and the $SiO_2$ is from about 0.60 to about 0.85, such as from about 0.60 to about 0.80, mole fraction.

Clause 13. The glass material according to any one of clauses 1 to 12, wherein the glass comprises BaO and $Na_2O$, and the sum of the BaO and the $Na_2O$ is from about 0.10 to about 0.33, such as from about 0.15 to about 0.33, mole fraction.

Clause 14. The glass material according to clause 13, wherein the sum of the BaO and the $Na_2O$ is from about 0.10 to about 0.25, such as from about 0.15 to about 0.25, mole fraction.

Clause 15. The glass material according to any one of clauses 1 to 14, wherein the sum of the $SiO_2$ and the $Na_2O$ is from about 0.65 to about 0.90 mole fraction.

Clause 16. The glass material according to clause 6, wherein:

the sum of the $SiO_2$ and the $Na_2O$ is from about 0.69 to about 0.90 mole fraction;
the glass comprises from about 0.05 to about 0.09 mole fraction of $Ta_2O_5$; and
the sum of the $Y_2O_3$, the BaO and the $Ta_2O_5$ is from about 0.10 to about 0.17 mole fraction.

Clause 17. The glass material according to clause 6, wherein:

the sum of the $SiO_2$ and the $Na_2O$ is from about 0.65 to about 0.80 mole fraction;
the glass comprises from about 0.05 to about 0.10 mole fraction of $Ta_2O_5$; and
the sum of the $Y_2O_3$, the BaO and the $Ta_2O_5$ is from about 0.18 to about 0.31 mole fraction.

Clause 18. The glass material according to clause 1, comprising:

from about 0.55 to about 0.82, such as from about 0.55 to about 0.75, mole fraction of $SiO_2$;
from about 0.03 to about 0.23, such as from about 0.05 to about 0.22, mole fraction of $Na_2O$;
from about 0.05 to about 0.12, such as from about 0.05 to about 0.08, mole fraction of: $Y_2O_3$, BaO, or a combination of $Y_2O_3$ and BaO; and
from about 0.05 to about 0.12, such as from about 0.05 to about 0.09, mole fraction of $Ta_2O_5$.

Clause 19. The glass material of clause 18 comprising:

from about 0.70 to about 0.73 mole fraction of $SiO_2$;
from about 0.16 to about 0.18 mole fraction of $Na_2O$;
from about 0.05 to about 0.7 mole fraction of $Y_2O_3$; and
from about 0.05 to about 0.7 mole fraction of $Ta_2O_5$.

Clause 20. The glass material of clause 19 comprising:

about 0.71 mole fraction of $SiO_2$;
about 0.17 mole fraction of $Na_2O$;
about 0.06 mole fraction of $Y_2O_3$; and
about 0.06 mole fraction of $Ta_2O_5$.

Clause 21. The glass material of clause 19 comprising:

about 0.72 mole fraction of $SiO_2$;
about 0.17 mole fraction of $Na_2O$;
about 0.05 mole fraction of $Y_2O_3$; and
about 0.06 mole fraction of $Ta_2O_5$.

Clause 22. The glass material of clause 18, further comprising up to 0.1 mole fraction of $B_2O_3$.

Clause 23. The glass material of clause 22 comprising:

from about 0.65 to about 0.82, such as about 0.73 to about 0.80, mole fraction of $SiO_2$;
from about 0.03 to about 0.23, such as about 0.03 to about 0.11, mole fraction of $Na_2O$;
from about 0.06 to about 0.12, such as about 0.07 to about 0.10, mole fraction of BaO;
from about 0.06 to about 0.12, such as about 0.07 to about 0.10, mole fraction of $Ta_2O_5$; and
from about 0.001 to about 0.015, such as about 0.001 to about 0.006, mole fraction of $B_2O_3$.

Clause 24. The glass material of clause 23 comprising:

about 0.69 mole fraction of $SiO_2$;
about 0.16 mole fraction of $Na_2O$;
about 0.07 mole fraction of BaO;
about 0.07 mole fraction of $Ta_2O_5$; and
about 0.012 mole fraction of $B_2O_3$.

Clause 25. The glass material of clause 23 comprising:

about 0.66 mole fraction of $SiO_2$;
about 0.20 mole fraction of $Na_2O$;
about 0.065 mole fraction of BaO;
about 0.06 mole fraction of $Ta_2O_5$; and
about 0.011 mole fraction of $B_2O_3$.

Clause 26. The glass material of clause 23 comprising:

about 0.73 mole fraction of $SiO_2$;

about 0.11 mole fraction of $Na_2O$;
about 0.07 mole fraction of $BaO$;
about 0.07 mole fraction of $Ta_2O_5$; and
about 0.011 mole fraction of $B_2O_3$.

Clause 27. The glass material of clause 23 comprising:

about 0.74 mole fraction of $SiO_2$;
about 0.09 mole fraction of $Na_2O$;
about 0.085 mole fraction of $BaO$;
about 0.085 mole fraction of $Ta_2O_5$; and
about 0.006 mole fraction of $B_2O_3$.

Clause 28. The glass material of clause 23 comprising:

about 0.79 mole fraction of $SiO_2$;
about 0.06 mole fraction of $Na_2O$;
about 0.08 mole fraction of $BaO$;
about 0.07 mole fraction of $Ta_2O_5$; and
about 0.003 mole fraction of $B_2O_3$.

Clause 29. The glass material of clause 23 comprising:

about 0.77 mole fraction of $SiO_2$;
about 0.031 mole fraction of $Na_2O$;
about 0.10 mole fraction of $BaO$;
about 0.096 mole fraction of $Ta_2O_5$; and
about 0.002 mole fraction of $B_2O_3$.

Clause 30. The glass material of clause 23 comprising:

about 0.80 mole fraction of $SiO_2$;
about 0.033 mole fraction of $Na_2O$;
about 0.082 mole fraction of $BaO$;
about 0.085 mole fraction of $Ta_2O_5$; and
about 0.001 mole fraction of $B_2O_3$.

Clause 31. The glass material of clause 22 comprising:

about 0.58 mole fraction of $SiO_2$;
about 0.20 mole fraction of $Na_2O$;
about 0.06 mole fraction of a combination of $Y_2O_3$ and $BaO$;
about 0.07 mole fraction of $Ta_2O_5$; and
about 0.09 mole fraction of $B_2O_3$.

Clause 32. The glass material of clause 31, comprising about 0.024 mole fraction of $Y_2O_3$ and about 0.035 mole fraction of $BaO$.

Clause 33. The glass material of any one of clauses 1 to 32, substantially lacking one or more of $Li_2O$, $Rb_2O$, $V_2O_5$, $ZnO$, $Fe_2O$, and fluoride.

Clause 34. The glass material of any one of clauses 1 to 33, wherein the glass material is a bulk glass.

Clause 35. The glass material of any one of clauses 1 to 33, wherein the glass material is an irregular microparticulate glass material.

Clause 36. The glass material according to clause 35, wherein the microparticulate glass material has an average diameter from about 15 $\mu$m to about 1200 $\mu$m.

Clause 37. The glass material according to any one of clauses 1 to 36, wherein the glass material exhibits a CT Radiopacity of more than 9,000 Hounsfield units at 120 kVp.

Clause 38. The glass material according to clauses 1 or 37, wherein the density of the glass material is from about 2.7 $g/cm^3$ to about 4.3 $g/cm^3$, such as from about 2.9 $g/cm^3$ to about 3.7 $g/cm^3$, or from about 3.3 $g/cm^3$ to about 3.5 $g/cm^3$.

Clause 39. A microparticulate glass material that is a substantially spherical microparticulate glass material obtained from spherodizing the irregular microparticulate glass material of clause 35 or 36.

Clause 40. The microparticulate glass material of clause 39 wherein spherodizing the irregular microparticulate glass material comprises re-melting the surface of the irregular microparticulate glass material in a flame, and allowing a substantially spherical drop to form.

Clause 41. The microparticulate glass material of clause 40, wherein the substantially spherical microparticulate glass material is obtained from conditioning the substantially spherical drops, such as by conditioning the flame-treated microparticulate glass material in a solution of calcium- and magnesium-free phosphate buffered saline (CMF-PBS), which CMF-PBS solution may be at a concentration of 0.2 g/mL.

Clause 42. The microparticulate glass material of clause 41, wherein the conditioning is for at least 12 hours at 80 °C.

Clause 43. The microparticulate glass material of clause 41 or 42, wherein the substantially spherical microparticulate glass material has a reduced amount of spherodizing-reaction products in comparison to the flame-treated microparticulate glass material.

Clause 44. The glass material of any one of clauses 1 to 33, wherein the glass material is a substantially spherical microparticulate glass material.

Clause 45. The microparticulate glass material according to any one of clauses 39 to 44, wherein the microparticulate glass material has an average diameter from about 15 $\mu$m to about 1200 $\mu$m.

Clause 46. The microparticulate glass material according to clause 45, wherein the microparticulate glass material has an average diameter from about 15 $\mu$m to about 1200 $\mu$m, such as from about 15 $\mu$m to about 35 $\mu$m; from about 40 $\mu$m to about 500 $\mu$m; from about 40 $\mu$m to about 300 $\mu$m; from about 300 $\mu$m to about 500 $\mu$m; from about 500 $\mu$m to about 700 $\mu$m; or from about 700 $\mu$m to about 1200 $\mu$m.

Clause 47. The microparticulate glass material according to any one of clauses 39 to 46, wherein the glass material exhibits a CT Radiopacity of more than 9,000 Hounsfield units at 120 kVp.

Clause 48. The microparticulate glass material according to any one of clauses 39 to 47 for radiography imaging, computerized tomography (CT) imaging, cone beam CT imaging, or fluoroscopy imaging.

Clause 49. The microparticulate glass material according to clause 48, wherein the glass material is compatible with positron emission tomography (PET), single-photon emission computed tomography (SPECT), magnetic resonance imaging (MRI), or any combination thereof.

Clause 50. A method comprising:

administering to a mammal, via intra-arterial or intravenous delivery, the microparticulate glass material according to any one of clauses 39 to 47, and
imaging the mammal, such as the liver of the mammal, by radiography imaging, computerized tomography (CT) imaging, cone beam CT imaging, or fluoroscopy imaging.

Clause 51. The method according to clause 50, wherein the mammal is a human.

Clause 52. The method according to clause 50 or 51, wherein the method comprises administering at least about 750 particles of the microparticulate glass material per gram of liver, such as about 1000 to about 5000 particles of the microparticulate glass material per gram of liver.

Clause 53. The method according to clause 52, wherein the method comprises administering to the human from about 1 million to about 7 million particles of the microparticulate glass material.

Clause 54. The method according to any one of clauses 50 to 52, wherein the method comprises intra-arterial delivery of the microparticulate glass material into a hepatic artery.

Clause 55. Use of the microparticulate glass material according to any one of clauses 39 to 47 for imaging a mammal, such as the liver of the mammal, by radiography imaging, computerized tomography (CT) imaging, cone beam CT imaging, or fluoroscopy imaging.

Clause 56. The use according to clause 55 for imaging the liver of the mammal, wherein the microparticulate glass material has been formulated to deliver at least about 750 particles of the microparticulate glass material per gram of the liver, such as about 1000 to about 5000 of the particles of the microparticulate glass material per gram of the liver.

Clause 57. A mixture of:

the microparticulate glass material according to any one of clauses 39 to 47; and
radioactive glass microparticles,
wherein the microparticulate glass material and the radioactive glass microparticles have substantially the same size.

Clause 58. The mixture according to clause 57, wherein the difference in average sizes of the microparticulate glass material and the radioactive glass microparticles is within 40% of the average of the two averages sizes.

Clause 59. The mixture according to clause 57, wherein the difference in average sizes of the microparticulate glass material and the radioactive glass microparticles is within 10% of the average of the two averages sizes.

Clause 60. The mixture according to any one of clauses 57 to 59, wherein the microparticulate glass material and the radioactive glass microparticles have substantially the same density.

Clause 61. The mixture according to clause 60, wherein the difference in particle densities of the microparticulate glass material and the radioactive glass microparticles is within 30%, and preferably within 15%, of the average of the two particle densities.

Clause 62. The mixture according to any one of clauses 57 to 61, wherein the microparticulate glass material comprises glass having:

from about 0.65 to about 0.82, such as about 0.73 to about 0.80, mole fraction of $SiO_2$;
from about 0.03 to about 0.23, such as about 0.03 to about 0.11, mole fraction of $Na_2O$;
from about 0.06 to about 0.12, such as about 0.07 to about 0.10, mole fraction of BaO;
from about 0.06 to about 0.12, such as about 0.07 to about 0.10, mole fraction of $Ta_2O_5$; and
from about 0.001 to about 0.015, such as about 0.001 to about 0.006, mole fraction of $B_2O_3$.

Clause 63. The mixture according to any one of clauses 57 to 62, wherein the radioactive glass microparticles is a yttrium oxide-aluminosilicate glass, preferably comprising about 40 wt% $SiO_2$, about 20 wt% $Al_2O_3$, and about 40 wt% $Y_2O_3$.

Clause 64. The mixture according to any one of clauses 57 to 63, wherein the radioactive glass microparticles are substantially spherical.

Clause 65. The mixture according to any one of clauses 57 to 64, wherein:

the microparticulate glass material comprises glass having:

from about 0.73 to about 0.80 mole fraction of $SiO_2$;
from about 0.03 to about 0.11 mole fraction of $Na_2O$;
from about 0.07 to about 0.10 mole fraction of BaO;
from about 0.07 to about 0.10 mole fraction of $Ta_2O_5$; and

from about 0.001 to about 0.006 mole fraction of $B_2O_3$; and

the radioactive glass microparticles are substantially spherical yttrium oxide-aluminosilicate glass particles comprising about 40 wt% $SiO_2$, about 20 wt% $Al_2O_3$, and about 40 wt% $Y_2O_3$.

Clause 66. A method for delivering radiation to a mammal, the method comprising: administering to a mammal, via intra-arterial or intravenous delivery, the mixture according to any one of clauses 57 to 65.

Clause 67. The method according to clause 66, further comprising imaging the mammal, such as the liver of the mammal, by radiography, computerized tomography (CT), cone beam CT, or fluoroscopy.

Clause 68. The method according to clause 67, further comprising calculating an absorbed dose of radiation to a tissue.

Clause 69. Use of a mixture according to any one of clauses 57 to 65 for delivering radiation to a mammal, wherein the mixture is formulated for intravenous or intra-arterial injection to the mammal.

Clause 70. A therapeutic or diagnostic composition comprising a mixture of:

(i) radioactive microparticles; and
(ii) non-radioactive microparticles comprising the glass material of any one of clauses 1 to 33.

Clause 71. The composition according to clause 70, wherein the radioactive microparticles and the non-radioactive microparticles have a difference in particle densities that is within 30%, and preferably within 15%, of the average of the two particle densities.

Clause 72. The composition according to clause 70 or 71, wherein the radioactive microparticles have an average diameter from about 10 to about 1200 microns, such as an average diameter from about 20 to about 40 microns; and the non-radioactive microparticles have an average size from about 10 to about 1200 microns, such as an average diameter from about 20 to about 40 microns.

Clause 73. The composition according to any one of clauses 70 to 72, wherein the radioactive microparticles and the non-radioactive microparticles have a difference in average sizes that is within 40% of the average of the two averages sizes.

Clause 74. The composition according to any one of clauses 70 to 73, wherein the radioactive microparticles and the non-radioactive microparticles have substantially the same resistance when flowing in a liquid through a conduit.

Clause 75. The composition according to any one of clauses 70 to 74, wherein the radioactive microparticles make up from about 10% to about 80%, such as about 25%, of the total mass of microparticles in the composition.

Clause 76. The composition according to any one of clauses 70 to 75, wherein the radioactive microparticles are diagnostic radioactive microparticles.

Clause 77. The composition according to any one of clauses 70 to 75, wherein the radioactive microparticles are therapeutic radioactive microparticles.

Clause 78. The composition according to clause 76, wherein the radioactive microparticles comprise one or more radioisotopes selected from the group consisting of: copper-67, holmium-166, indium-111, iodine-131, lutetium-177, molybdenum-99, phosphorus-32, rubidium-82, technicium-99m, and thallium-201.

Clause 79. The composition according to clause 77, wherein the radioactive microparticles comprise one or more radioisotopes selected from the group consisting of: actinium-225, bismuth-213, copper-67, indium-111, iodine-131, iodine-125, gadolinium-157, holmium-166, lead-212, lutetium-177, palladium-103, phosphorus-32, radium-223, rhenium-186, rhenium-188, samarium-153, strontium-89, and tungsten-188.

Clause 80. The composition according to any one of clauses 70 to 75, wherein the radioactive microparticles comprise about 40 wt% $Y_2O_3$, about 20 wt% $Al_2O_3$, and about 40 wt% $SiO_2$, wherein at least a portion of the yttrium is yttrium-90.

Clause 81. The composition according to any one of clauses 70 to 80, wherein the non-radioactive microparticles comprise the glass material according to any one of clauses 18 to 33.

Clause 82. The composition according to any one of clauses 70 to 81, wherein the radioactive microparticles are substantially spherical, the non-radioactive microparticles are substantially spherical, or both.

Clause 83. A method comprising:
administering a composition according to any one of clauses 70 to 82 to a patient, wherein the administration is: by intravascular delivery, intra-peritoneal delivery, or percutaneous delivery.

Clause 84. A delivery device for intravascular delivery, intra-peritoneal delivery, or percutaneous delivery of a mixture of radioactive microparticles and non-radioactive microparticles to a patient, the delivery device being fluidly coupleable to a mixing and transport medium, the delivery device comprising:

> a fluid inlet fluidly coupleable to the mixing and transport medium;
> a fluid outlet;
> a fluid mixer fluidly coupled to the fluid inlet and to the fluid outlet;
> a source of radioactive microparticles fluidly coupled to the fluid mixer; and
> a source of non-radioactive microparticles fluidly coupled to the fluid mixer, wherein the non-radioactive microparticles comprise the glass material of any one of clauses 1 to 33;
> wherein the source of the radioactive microparticles is distinct from the source of non-radioactive microparticles; and
> wherein the fluid mixer mixes radioactive microparticles with the non-radioactive microparticles, and delivers the mixture of radioactive and non-radioactive microparticles out of the fluid outlet utilizing the mixing and transport medium.

Clause 85. A delivery device for intravascular delivery, intra-peritoneal delivery, or percutaneous delivery of a mixture of radioactive microparticles and non-radioactive microparticles to a patient, the delivery device comprising:

> at least one fluid inlet fluidly coupleable to a transport medium;
> a source of radioactive microparticles fluidly coupled to the at least one fluid inlet;
> a source of non-radioactive microparticles fluidly coupled to the at least one fluid inlet, wherein the non-radioactive microparticles comprise the glass material of any one of clauses 1 to 33;
> a first fluid outlet fluidly coupled to the source of the radioactive microparticles; and
> a second fluid outlet fluidly coupled to the source of non-radioactive microparticles;
> wherein the source of the radioactive microparticles is distinct from the source of non-radioactive microparticles.

Clause 86. The delivery device according to clause 85, wherein the delivery device delivers of the radioactive microparticles and the non-radioactive microparticles in a single treatment session.

Clause 87. The delivery device according to clause 85 or 86, wherein the first fluid outlet and the second fluid outlet are proximate to each other.

Clause 88. A method comprising:

> mixing (i) a first population of radioactive microparticles and (ii) a second population of non-radioactive microparticles, wherein the second population of non-radioactive microparticles comprises microparticles comprising the glass material of any one of clauses 1 to 33;
> administering a therapeutically or diagnostically relevant amount of the mixture to a patient.

Clause 89. The method according to clause 88, wherein the radioactive microparticles make up from about 10% to about 80%, such as about 25%, of the total mass of microparticles in the mixture.

Clause 90. The method according to clause 88 or 89, wherein the administration is by intravascular delivery, intra-peritoneal delivery, or percutaneous delivery.

Clause 91. A method of administering a therapeutically or diagnostically relevant amount of microparticles to a patient, the method comprising:

administering to the patient non-radioactive microparticles comprising the glass material of any one of clauses 1 to 33; and
administering radioactive microparticles to the patient without first detecting the non-radioactive microparticles; wherein the administration is by intravascular delivery, intra-peritoneal delivery, or percutaneous delivery; and wherein the route of administration of the non-radioactive microparticles is the same as the route of administration of the radioactive microparticles.

Clause 92. The method according to clause 91, wherein the method comprises concurrent administration of the non-radioactive and the radioactive microparticles.

Clause 93. The method according to clause 91, wherein the method comprises sequential administration of the non-radioactive and the radioactive microparticles.

Clause 94. A method of administering a therapeutically or diagnostically relevant amount of microparticles to a patient, the method comprising:

administering radioactive microparticles to the patient; and
administering to the patient non-radioactive microparticles comprising the glass material of any one of clauses 1 to 33, without first detecting the radioactive microparticles;
wherein the administration is by intravascular delivery, intra-peritoneal delivery, or percutaneous delivery; and wherein the route of administration of the non-radioactive microparticles is the same as the route of administration of the radioactive microparticles.

Clause 95. The method according to clause 94, wherein the method comprises concurrent administration of the radioactive and the non-radioactive microparticles.

Clause 96. The method according to clause 94, wherein the method comprises sequential administration of the radioactive and the non-radioactive microparticles.

Clause 97. A method of administering a therapeutically or diagnostically relevant amount of microparticles, the method comprising:
concurrent administration of (i) a first population of radioactive microparticles and (ii) a second population of non-radioactive microparticles to a patient, wherein the second population of non-radioactive microparticles comprises microparticles comprising the glass material of any one of clauses 1 to 33.

Clause 98. The method according to clause 97, wherein the first population of radioactive microparticles is distinct from the second population of non-radioactive microparticles.

Clause 99. The method according to clause 98, wherein the first population of radioactive microparticles and the second population of non-radioactive microparticles are administered as a mixture.

Clause 100. A method of administering a therapeutically or diagnostically relevant amount of microparticles, the method comprising:
sequential administration in a single treatment session of non-radioactive microparticles comprising the glass material of any one of clauses 1 to 33, and of radioactive microparticles to a patient.

Clause 101. A method comprising
sequential administration to a patient of

(i) therapeutically radioactive microparticles, and then
(ii) non-radioactive microparticles comprising the glass material of any one of clauses 1 to 33.

Clause 102. The method according to clause 93, 96, 100 or 101 wherein the sequential administration comprises intermittent administration of the non-radioactive microparticles and the radioactive microparticles.

Clause 103. The method according to clause 102, wherein the intermittent administration comprises alternating administration of the non-radioactive microparticles and the radioactive microparticles.

Clause 104. The method according to clause 93 or 100, wherein the sequential administration comprises administration of all of the non-radioactive microparticles before administration of any of the radioactive microparticles.

Clause 105. The method according to clause 96 or 101, wherein the sequential administration comprises administration of all of the radioactive microparticles before administration of any of the non-radioactive microparticles.

Clause 106. The method according to any one of clauses 88 to 104, wherein the method delivers a therapeutically relevant amount of radiation to the patient.

Clause 107. The method according to any one of clauses 88 to 105, wherein the method delivers a diagnostically relevant amount of the non-radioactive microparticles to the patient.

Clause 108. The method according to any one of clauses 97 to 107, wherein the administration is by intravascular delivery, intra-peritoneal delivery, or percutaneous delivery.

Clause 109. The method according to any one of clauses 88 to 108, wherein about 10% to about 80%, such as about 25%, of the total mass of microparticles delivered are radioactive microparticles.

Clause 110. The mixture according to any one of clauses 57 to 65, the composition according to any one of clauses 70 to 82, the delivery device according to any one of clauses 84 to 87, or the method according to any one of clauses 83 and 88 to 109,

> wherein the radioactive microparticles and the non-radioactive microparticles have substantially the same resistance when flowing in a liquid through a conduit; and/or
> wherein the radioactive microparticles and the non-radioactive microparticles are suitable for vascular embolization.

Since the above description provides examples, it will be appreciated that modifications and variations can be effected to the particular examples by those of skill in the art. Accordingly, the scope of the claims should not be limited by the particular examples set forth herein, but should be construed in a manner consistent with the specification as a whole.

**Claims**

1. A glass material, wherein the glass comprises:

   from about 0.55 to about 0.85 mole fraction of $SiO_2$;
   from about 0.01 to about 0.23 mole fraction of $Na_2O$, $K_2O$, or a combination of $Na_2O$ and $K_2O$;
   from about 0.05 to about 0.28 mole fraction of: $Y_2O_3$, BaO, or a combination of $Y_2O_3$ and BaO; and
   $Ta_2O_5$ in an amount up to about 0.12 mole fraction of $Ta_2O_5$,
   wherein the sum of the $Y_2O_3$, the BaO and the $Ta_2O_5$ is from about 0.10 to about 0.31 mole fraction; and
   wherein the glass substantially lacks $Li_2O$, $Rb_2O$, $V_2O_5$, ZnO, $Fe_2O$, and fluoride.

2. The glass material according to claim 1, wherein the glass comprises from about 0.05 to about 0.12, such as from about 0.05 to about 0.10, mole fraction of $Ta_2O_5$.

3. A glass material, wherein the glass comprises:

   from about 0.55 to about 0.85 mole fraction of $SiO_2$;
   from about 0.01 to about 0.23 mole fraction of $Na_2O$, $K_2O$, or a combination of $Na_2O$ and $K_2O$;
   from about 0.05 to about 0.28 mole fraction of: $Y_2O_3$, BaO, or a combination of $Y_2O_3$ and BaO;
   $B_2O_3$ in an amount up to 0.20 mole fraction of $B_2O_3$; and
   optionally $Ta_2O_5$,
   wherein the glass comprises BaO and $Na_2O$, and the sum of the BaO and the $Na_2O$ is from about 0.10 to about 0.33, such as from about 0.15 to about 0.33, mole fraction; preferably wherein the sum of the BaO and the $Na_2O$ is

from about 0.10 to about 0.25, such as from about 0.15 to about 0.25, mole fraction;
wherein the sum of the $Y_2O_3$, the BaO and the optional $Ta_2O_5$ is from about 0.10 to about 0.31 mole fraction; and
wherein the glass substantially lacks $Li_2O$, $Rb_2O$, $V_2O_5$, ZnO, $Fe_2O$, and fluoride.

4. The glass material according to claim 3, wherein the sum of the $B_2O_3$ and the $SiO_2$ is from about 0.60 to about 0.85, such as from about 0.60 to about 0.80, mole fraction.

5. The glass material according to any one of claims 1 to 4, wherein the glass comprises from about 0.03 to about 0.23 mole fraction of $Na_2O$, $K_2O$, or a combination of $Na_2O$ and $K_2O$; preferably wherein the glass comprises from about 0.05 to about 0.23 mole fraction of $Na_2O$, $K_2O$, or a combination of $Na_2O$ and $K_2O$.

6. The glass material according to any one of claims 1 to 5, wherein the glass comprises from about 0.55 to about 0.82, preferably from about 0.55 to about 0.80, mole fraction of $SiO_2$.

7. The glass material according to any one of claims 1 to 4, wherein the glass comprises:

   from about 0.55 to about 0.80 mole fraction of $SiO_2$; and
   from about 0.05 to about 0.22 mole fraction of $Na_2O$, $K_2O$, or a combination of $Na_2O$ and $K_2O$.

8. The glass material according to any one of claims 1 to 7, wherein the sum of the $Y_2O_3$, the BaO and any $Ta_2O_5$ is from about 0.10 to about 0.25 mole fraction, such as from about 0.10 to about 0.15 mole fraction.

9. The glass material according to any one of claims 1 to 8, wherein the glass comprises from about 0.05 to about 0.12, such as from about 0.05 to about 0.08, mole fraction of $Y_2O_3$, BaO, or a combination of $Y_2O_3$ and BaO.

10. The glass material according to claim 1 or 2, or according to any one of claims 5 to 9 when dependent from claim 1 or 2, wherein the glass comprises BaO and $Na_2O$, and the sum of the BaO and the $Na_2O$ is from about 0.10 to about 0.33, such as from about 0.15 to about 0.33, mole fraction; preferably wherein the sum of the BaO and the $Na_2O$ is from about 0.10 to about 0.25, such as from about 0.15 to about 0.25, mole fraction.

11. The glass material according to any one of claims 1 to 10, wherein the sum of the $SiO_2$ and the $Na_2O$ is from about 0.65 to about 0.90 mole fraction.

12. The glass material according to claim 1, wherein:

    (i) the glass comprises

       from about 0.55 to about 0.80 mole fraction of $SiO_2$; and
       from about 0.05 to about 0.22 mole fraction of $Na_2O$, $K_2O$, or a combination of $Na_2O$ and $K_2O$; and
       the sum of the $SiO_2$ and the $Na_2O$ is from about 0.69 to about 0.90 mole fraction;
       the glass comprises from about 0.05 to about 0.09 mole fraction of $Ta_2O_5$; and
       the sum of the $Y_2O_3$, the BaO and the $Ta_2O_5$ is from about 0.10 to about 0.17 mole fraction; or

    (ii) the glass comprises

       from about 0.55 to about 0.80 mole fraction of $SiO_2$; and
       from about 0.05 to about 0.22 mole fraction of $Na_2O$, $K_2O$, or a combination of $Na_2O$ and $K_2O$; and
       the sum of the $SiO_2$ and the $Na_2O$ is from about 0.65 to about 0.80 mole fraction;
       the glass comprises from about 0.05 to about 0.10 mole fraction of $Ta_2O_5$; and
       the sum of the $Y_2O_3$, the BaO and the $Ta_2O_5$ is from about 0.18 to about 0.31 mole fraction.

13. The glass material according to claim 1, comprising:

    from about 0.55 to about 0.82, such as from about 0.55 to about 0.75, mole fraction of $SiO_2$;
    from about 0.03 to about 0.23, such as from about 0.05 to about 0.22, mole fraction of $Na_2O$;
    from about 0.05 to about 0.12, such as from about 0.05 to about 0.08, mole fraction of: $Y_2O_3$, BaO, or a combination of $Y_2O_3$ and BaO; and
    from about 0.05 to about 0.12, such as from about 0.05 to about 0.09, mole fraction of $Ta_2O_5$.

**14.** The glass material of claim 13 comprising:

from about 0.70 to about 0.73 mole fraction of $SiO_2$;
from about 0.16 to about 0.18 mole fraction of $Na_2O$;
from about 0.05 to about 0.7 mole fraction of $Y_2O_3$; and
from about 0.05 to about 0.7 mole fraction of $Ta_2O_5$.

**15.** The glass material of claim 3 comprising:

from about 0.65 to about 0.82, such as about 0.73 to about 0.80, mole fraction of $SiO_2$;
from about 0.03 to about 0.23, such as about 0.03 to about 0.11, mole fraction of $Na_2O$;
from about 0.06 to about 0.12, such as about 0.07 to about 0.10, mole fraction of $BaO$;
from about 0.06 to about 0.12, such as about 0.07 to about 0.10, mole fraction of $Ta_2O_5$; and
from about 0.001 to about 0.015, such as about 0.001 to about 0.006, mole fraction of $B_2O_3$.

**Fig. 1**

**Fig. 2A**

10.0kV 12.0mm x250 SE(L)  200um   10.0kV 11.8mm x1.00k SE(L)  50.0um

**Fig. 2B**

1.5kV 12.8mm x250 SE(L)  200um   1.5kV 12.8mm x1.00k SE(L)  50.0um

**Fig. 2C**

1.5kV 12.3mm x250 SE(L)  200um   1.5kV 12.3mm x1.00k SE(L)  50.0um

**Fig. 2D**

Fig. 3

EP 4 763 817 A2

Fig. 4

EP 4 763 817 A2

Fig. 5

40

**Fig. 6**

Fig. 7

EP 4 763 817 A2

**Fig. 8**

EP 4 763 817 A2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 62855285

- US 4789501 A

### Non-patent literature cited in the description

- **PARMAR et al.** Size Dependent Heating Efficiency of Iron Oxide Single Domain Nanoparticles. *Procedia Engineering*, 2015, vol. 102, 527-533